# EUROPÄISCHE PATENTSCHRIFT

(11) **EP 2 260 303 B1**
(45) Veröffentlichungstag und Bekanntmachung des Hinweises auf die Patenterteilung: **08.01.2014**
(21) Anmeldenummer: 09715706.9
(22) Anmeldetag: 02.03.2009
(51) Int. Cl.: G01N 33/574

(54) **BIOMARKER FÜR DIE DIAGNOSE VON HIRNTUMOR**
BIOMARKER FOR THE DIAGNOSIS OF A BRAIN TUMOR
BIOMARQUEUR SERVANT À DIAGNOSTIQUER UNE TUMEUR DU CERVEAU

(30) Priorität: 29.02.2008 DE 102008011850
(43) Veröffentlichungstag der Anmeldung: 15.12.2010
(73) Patentinhaber: Meyer, Helmut E., 45661 Recklinghausen (DE); Grzendowski, Michael, 47445 Moers (DE); Stühler, Kai, 50733 Köln (DE); Reifenberger, Guido, 40591 Düsseldorf (DE); Wolter, Marietta, 40591 Düsseldorf (DE); Riemenschneider, Markus, 40225 Düsseldorf (DE)
(72) Erfinder: Meyer, Helmut E., 45661 Recklinghausen (DE); Grzendowski, Michael, 47445 Moers (DE); Stühler, Kai, 50733 Köln (DE); Reifenberger, Guido, 40591 Düsseldorf (DE); Wolter, Marietta, 40591 Düsseldorf (DE); Riemenschneider, Markus, 40225 Düsseldorf (DE)
(74) Vertreter: Simandi, Claus
(86) Internationale Anmeldenummer: PCT/DE2009/000275
(87) Internationale Veröffentlichungsnummer: WO 2009/106065

(56) Entgegenhaltungen:
- EP-A- 1 775 590
- WO-A-02/082076
- WO-A-2004/016758
- WO-A-2007/039963
- US-A1- 2005 014 292
- US-A1- 2007 141 066
- LI J ET AL: 'Proteomic profiling distinguishes astrocytomas and identifies differential tumor markers.' NEUROLOGY 14 MAR 2006 LNKD- PUBMED:16534112 Bd. 66, Nr. 5, 14 März 2006, Seiten 733 - 736, XP008135108 ISSN: 1526-632X
- KHALIL ASHRAF A ET AL: "Biomarker discovery: A proteomic approach for brain cancer profiling", CANCER SCIENCE, vol. 98, no. 2, February 2007 (2007-02), pages 201-213, ISSN: 1347-9032
- MUSTAFA DANA A N ET AL: "Identification of glioma neovascularization-related proteins by using MALDI-FTMS and Nano-LC fractionation to microdissected tumor vessels", MOLECULAR & CELLULAR PROTEOMICS, AMERICAN SOCIETY FOR BIOCHEMISTRY AND MOLECULAR BIOLOGY, INC, US, vol. 6, no. 7, 1 July 2007 (2007-07-01), pages 1147-1157, XP002474817, ISSN: 1535-9476, DOI: DOI:10.1074/MCP.M600295-MCP200
- HIRATSUKA M ET AL: "Proteomics-based identification of differentially expressed genes in human gliomas: down-regulation of SIRT2 gene", BIOCHEMICAL AND BIOPHYSICAL RESEARCH COMMUNICATIONS, ACADEMIC PRESS INC. ORLANDO, FL, US, vol. 309, no. 3, 26 September 2003 (2003-09-26), pages 558-566, XP004453635, ISSN: 0006-291X, DOI: DOI:10.1016/J.BBRC.2003.08.029

### SEQUENCE LISTING

<110> Meyer, Helmut et al
<120> Biomarker für die Diagnose von Hirntumor
<130> Meyer 32-11WO
<160> 24
<170> PatentIn version 3.3
<210> 1
   <211> 316
   <212> PRT
   <213> Homo sapiens
<400> 1
   Met Ala Ser Arg Leu Leu Leu Asn Asn Gly Ala Lys Met Pro Ile Leu
   1               5                   10                  15
   Gly Leu Gly Thr Trp Lys Ser Pro Pro Gly Gln Val Thr Glu Ala Val
               20                  25                  30
   Lys Val Ala Ile Asp Val Gly Tyr Arg His Ile Asp Cys Ala His Val
           35                  40                  45
   Tyr Gln Asn Glu Asn Glu Val Gly Val Ala Ile Gln Glu Lys Leu Arg
       50                  55                  60
   Glu Gln Val Val Lys Arg Glu Glu Leu Phe Ile Val Ser Lys Leu Trp
   65                  70                  75                  80
   Cys Thr Tyr His Glu Lys Gly Leu Val Lys Gly Ala Cys Gln Lys Thr
                   85                  90                  95
   Leu Ser Asp Leu Lys Leu Asp Tyr Leu Asp Leu Tyr Leu Ile His Trp
               100                 105                 110
   Pro Thr Gly Phe Lys Pro Gly Lys Glu Phe Phe Pro Leu Asp Glu Ser
           115                 120                 125
   Gly Asn Val Val Pro Ser Asp Thr Asn Ile Leu Asp Thr Trp Ala Ala
       130                 135                 140
   Met Glu Glu Leu Val Asp Glu Gly Leu Val Lys Ala Ile Gly Ile Ser
   145                 150                 155                 160
   Asn Phe Asn His Leu Gln Val Glu Met Ile Leu Asn Lys Pro Gly Leu
                   165                 170                 175
   Lys Tyr Lys Pro Ala Val Asn Gln Ile Glu Cys His Pro Tyr Leu Thr
               180                 185                 190
   Gln Glu Lys Leu Ile Gln Tyr Cys Gln Ser Lys Gly Ile Val Val Thr
   195 200 205
   Ala Tyr Ser Pro Leu Gly Ser Pro Asp Arg Pro Trp Ala Lys Pro Glu
   210 215 220
   Asp Pro Ser Leu Leu Glu Asp Pro Arg Ile Lys Ala Ile Ala Ala Lys
   225 230 235 240
   His Asn Lys Thr Thr Ala Gln Val Leu Ile Arg Phe Pro Met Gln Arg
   245 250 255
   Asn Leu Val Val Ile Pro Lys Ser Val Thr Pro Glu Arg Ile Ala Glu
   260 265 270
   Asn Phe Lys Val Phe Asp Phe Glu Leu Ser Ser Gln Asp Met Thr Thr
   275 280 285
   Leu Leu Ser Tyr Asn Arg Asn Trp Arg Val Cys Ala Leu Leu Ser Cys
   290 295 300
   Thr Ser His Lys Asp Tyr Pro Phe His Glu Glu Phe
   305 310 315
<210> 2
   <211> 175
   <212> PRT
   <213> Homo sapiens
<400> 2
<210> 3
   <211> 434
   <212> PRT
   <213> Homo sapiens
<400> 3
   Met Ser Ile Leu Lys Ile His Ala Arg Glu Ile Phe Asp Ser Arg Gly
   1 5 10 15
   Asn Pro Thr Val Glu Val Asp Leu Phe Thr Ser Lys Gly Leu Phe Arg
   20 25 30
   Ala Ala Val Pro Ser Gly Ala Ser Thr Gly Ile Tyr Glu Ala Leu Glu
   35 40 45
   Leu Arg Asp Asn Asp Lys Thr Arg Tyr Met Gly Lys Gly Val Ser Lys
   50 55 60
   Ala Val Glu His Ile Asn Lys Thr Ile Ala Pro Ala Leu Val Ser Lys
   65 70 75 80
   Lys Leu Asn Val Thr Glu Gln Glu Lys Ile Asp Lys Leu Met Ile Glu
   85 90 95
   Met Asp Gly Thr Glu Asn Lys Ser Lys Phe Gly Ala Asn Ala Ile Leu
   100 105 110
   Gly Val Ser Leu Ala Val Cys Lys Ala Gly Ala Val Glu Lys Gly Val
   115 120 125
   Pro Leu Tyr Arg His Ile Ala Asp Leu Ala Gly Asn Ser Glu Val Ile
   130 135 140
   Leu Pro Val Pro Ala Phe Asn Val Ile Asn Gly Gly Ser His Ala Gly
   145 150 155 160
   Asn Lys Leu Ala Met Gln Glu Phe Met Ile Leu Pro Val Gly Ala Ala
   165 170 175
   Asn Phe Arg Glu Ala Met Arg Ile Gly Ala Glu Val Tyr His Asn Leu
   180 185 190
   Lys Asn Val Ile Lys Glu Lys Tyr Gly Lys Asp Ala Thr Asn Val Gly
           195                 200                 205
   Asp Glu Gly Gly Phe Ala Pro Asn Ile Leu Glu Asn Lys Glu Gly Leu
       210                 215                 220
   Glu Leu Leu Lys Thr Ala Ile Gly Lys Ala Gly Tyr Thr Asp Lys Val
   225                 230                 235                 240
   Val Ile Gly Met Asp Val Ala Ala Ser Glu Phe Phe Arg Ser Gly Lys
                   245                 250                 255
   Tyr Asp Leu Asp Phe Lys Ser Pro Asp Asp Pro Ser Arg Tyr Ile Ser
               260                 265                 270
   Pro Asp Gln Leu Ala Asp Leu Tyr Lys Ser Phe Ile Lys Asp Tyr Pro
           275                 280                 285
   Val Val Ser Ile Glu Asp Pro Phe Asp Gln Asp Asp Trp Gly Ala Trp
       290                 295                 300
   Gln Lys Phe Thr Ala Ser Ala Gly Ile Gln Val Val Gly Asp Asp Leu
   305                 310                 315                 320
   Thr Val Thr Asn Pro Lys Arg Ile Ala Lys Ala Val Asn Glu Lys Ser
                   325                 330                 335
   Cys Asn Cys Leu Leu Leu Lys Val Asn Gln Ile Gly Ser Val Thr Glu
               340                 345                 350
   Ser Leu Gln Ala Cys Lys Leu Ala Gln Ala Asn Gly Trp Gly Val Met
           355                 360                 365
   Val Ser His Arg Ser Gly Glu Thr Glu Asp Thr Phe Ile Ala Asp Leu
       370                 375                 380
   Val Val Gly Leu Cys Thr Gly Gln Ile Lys Thr Gly Ala Pro Cys Arg
   385                 390                 395                 400
   Ser Glu Arg Leu Ala Lys Tyr Asn Gln Leu Leu Arg Ile Glu Glu Glu
                   405                 410                 415
   Leu Gly Ser Lys Ala Lys Phe Ala Gly Arg Asn Phe Arg Asn Pro Leu
               420                 425                 430
   Ala Lys
<210> 4
   <211> 346
   <212> PRT
   <213> Homo sapiens
<400> 4
   Met Ala Met Val Ser Glu Phe Leu Lys Gln Ala Trp Phe Ile Glu Asn
   1               5                   10                  15
   Glu Glu Gln Glu Tyr Val Gln Thr Val Lys Ser Ser Lys Gly Gly Pro
               20                  25                  30
   Gly Ser Ala Val Ser Pro Tyr Pro Thr Phe Asn Pro Ser Ser Asp Val
           35                  40                  45
   Ala Ala Leu His Lys Ala Ile Met Val Lys Gly Val Asp Glu Ala Thr
       50                  55                  60
   Ile Ile Asp Ile Leu Thr Lys Arg Asn Asn Ala Gln Arg Gln Gln Ile
   65                  70                  75                  80
   Lys Ala Ala Tyr Leu Gln Glu Thr Gly Lys Pro Leu Asp Glu Thr Leu
                   85                  90                  95
   Lys Lys Ala Leu Thr Gly His Leu Glu Glu Val Val Leu Ala Leu Leu
               100                 105                 110
   Lys Thr Pro Ala Gln Phe Asp Ala Asp Glu Leu Arg Ala Ala Met Lys
           115                 120                 125
   Gly Leu Gly Thr Asp Glu Asp Thr Leu Ile Glu Ile Leu Ala Ser Arg
       130                 135                 140
   Thr Asn Lys Glu Ile Arg Asp Ile Asn Arg Val Tyr Arg Glu Glu Leu
   145                 150                 155                 160
   Lys Arg Asp Leu Ala Lys Asp Ile Thr Ser Asp Thr Ser Gly Asp Phe
                   165                 170                 175
   Arg Asn Ala Leu Leu Ser Leu Ala Lys Gly Asp Arg Ser Glu Asp Phe
               180                 185                 190
   Gly Val Asn Glu Asp Leu Ala Asp Ser Asp Ala Arg Ala Leu Tyr Glu
           195                 200                 205
   Ala Gly Glu Arg Arg Lys Gly Thr Asp Val Asn Val Phe Asn Thr Ile
       210                 215                 220
   Leu Thr Thr Arg Ser Tyr Pro Gln Leu Arg Arg Val Phe Gln Lys Tyr
   225                 230                 235                 240
   Thr Lys Tyr Ser Lys His Asp Met Asn Lys Val Leu Asp Leu Glu Leu
                   245                 250                 255
   Lys Gly Asp Ile Glu Lys Cys Leu Thr Ala Ile Val Lys Cys Ala Thr
   260 265 270
   Ser Lys Pro Ala Phe Phe Ala Glu Lys Leu His Gln Ala Met Lys Gly
   275 280 285
   Val Gly Thr Arg His Lys Ala Leu Ile Arg Ile Met Val Ser Arg Ser
   290 295 300
   Glu Ile Asp Met Asn Asp Ile Lys Ala Phe Tyr Gln Lys Met Tyr Gly
   305 310 315 320
   Ile Ser Leu Cys Gln Ala Ile Leu Asp Glu Thr Lys Gly Asp Tyr Glu
   325 330 335
   Lys Ile Leu Val Ala Leu Cys Gly Gly Asn
   340 345
<210> 5
   <211> 320
   <212> PRT
   <213> Homo sapiens
<400> 5
   Met Ala Gln Val Leu Arg Gly Thr Val Thr Asp Phe Pro Gly Phe Asp
   1               5                   10                  15
   Glu Arg Ala Asp Ala Glu Thr Leu Arg Lys Ala Met Lys Gly Leu Gly
               20                  25                  30
   Thr Asp Glu Glu Ser Ile Leu Thr Leu Leu Thr Ser Arg Ser Asn Ala
           35                  40                  45
   Gln Arg Gln Glu Ile Ser Ala Ala Phe Lys Thr Leu Phe Gly Arg Asp
       50                  55                  60
   Leu Leu Asp Asp Leu Lys Ser Glu Leu Thr Gly Lys Phe Glu Lys Leu
   65                  70                  75                  80
   Ile Val Ala Leu Met Lys Pro Ser Arg Leu Tyr Asp Ala Tyr Glu Leu
                   85                  90                  95
   Lys His Ala Leu Lys Gly Ala Gly Thr Asn Glu Lys Val Leu Thr Glu
               100                 105                 110
   Ile Ile Ala Ser Arg Thr Pro Glu Glu Leu Arg Ala Ile Lys Gln Val
           115                 120                 125
   Tyr Glu Glu Glu Tyr Gly Ser Ser Leu Glu Asp Asp Val Val Gly Asp
       130                 135                 140
   Thr Ser Gly Tyr Tyr Gln Arg Met Leu Val Val Leu Leu Gln Ala Asn
   145 150 155 160
   Arg Asp Pro Asp Ala Gly Ile Asp Glu Ala Gln Val Glu Gln Asp Ala
   165 170 175
   Gln Ala Leu Phe Gln Ala Gly Glu Leu Lys Trp Gly Thr Asp Glu Glu
   180 185 190
   Lys Phe Ile Thr Ile Phe Gly Thr Arg Ser Val Ser His Leu Arg Lys
   195 200 205
   Val Phe Asp Lys Tyr Met Thr Ile Ser Gly Phe Gln Ile Glu Glu Thr
   210 215 220
   Ile Asp Arg Glu Thr Ser Gly Asn Leu Glu Gln Leu Leu Leu Ala Val
   225 230 235 240
   Val Lys Ser Ile Arg Ser Ile Pro Ala Tyr Leu Ala Glu Thr Leu Tyr
   245 250 255
   Tyr Ala Met Lys Gly Ala Gly Thr Asp Asp His Thr Leu Ile Arg Val
   260 265 270
   Met Val Ser Arg Ser Glu Ile Asp Leu Phe Asn Ile Arg Lys Glu Phe
   275 280 285
   Arg Lys Asn Phe Ala Thr Ser Leu Tyr Ser Met Ile Lys Gly Asp Thr
   290 295 300
   Ser Gly Asp Tyr Lys Lys Ala Leu Leu Leu Leu Cys Gly Glu Asp Asp
   305 310 315 320
<210> 6
   <211> 241
   <212> PRT
   <213> Homo sapiens
<400> 6
   Met Ala Glu Glu Gln Pro Gln Val Glu Leu Phe Val Lys Ala Gly Ser
   1               5                   10                  15
   Asp Gly Ala Lys Ile Gly Asn Cys Pro Phe Ser Gln Arg Leu Phe Met
               20                  25                  30
   Val Leu Trp Leu Lys Gly Val Thr Phe Asn Val Thr Thr Val Asp Thr
           35                  40                  45
   Lys Arg Arg Thr Glu Thr Val Gln Lys Leu Cys Pro Gly Gly Gln Leu
       50                  55                  60
   Pro Phe Leu Leu Tyr Gly Thr Glu Val His Thr Asp Thr Asn Lys Ile
   65                  70                  75                  80
   Glu Glu Phe Leu Glu Ala Val Leu Cys Pro Pro Arg Tyr Pro Lys Leu
                   85                  90                  95
   Ala Ala Leu Asn Pro Glu Ser Asn Thr Ala Gly Leu Asp Ile Phe Ala
               100                 105                 110
   Lys Phe Ser Ala Tyr Ile Lys Asn Ser Asn Pro Ala Leu Asn Asp Asn
           115                 120                 125
   Leu Glu Lys Gly Leu Leu Lys Ala Leu Lys Val Leu Asp Asn Tyr Leu
       130                 135                 140
   Thr Ser Pro Leu Pro Glu Glu Val Asp Glu Thr Ser Ala Glu Asp Glu
   145                 150                 155                 160
   Gly Val Ser Gln Arg Lys Phe Leu Asp Gly Asn Glu Leu Thr Leu Ala
                   165                 170                 175
   Asp Cys Asn Leu Leu Pro Lys Leu His Ile Val Gln Val Val Cys Lys
               180                 185                 190
   Lys Tyr Arg Gly Phe Thr Ile Pro Glu Ala Phe Arg Gly Val His Arg
           195                 200                 205
   Tyr Leu Ser Asn Ala Tyr Ala Arg Glu Glu Phe Ala Ser Thr Cys Pro
       210                 215                 220
   Asp Asp Glu Glu Ile Glu Leu Ala Tyr Glu Gln Val Ala Lys Ala Leu
   225                 230                 235                 240
   Lys
<210> 7
   <211> 340
   <212> PRT
   <213> Homo sapiens
<400> 7
   Met Gly Lys Asp Tyr Tyr Gln Thr Leu Gly Leu Ala Arg Gly Ala Ser
   1               5                   10                  15
   Asp Glu Glu Ile Lys Arg Ala Tyr Arg Arg Gln Ala Leu Arg Tyr His
               20                  25                  30
   Pro Asp Lys Asn Lys Glu Pro Gly Ala Glu Glu Lys Phe Lys Glu Ile
           35                  40                  45
   Ala Glu Ala Tyr Asp Val Leu Ser Asp Pro Arg Lys Arg Glu Ile Phe
       50                  55                  60
   Asp Arg Tyr Gly Glu Glu Gly Leu Lys Gly Ser Gly Pro Ser Gly Gly
   65 70 75 80
   Ser Gly Gly Gly Ala Asn Gly Thr Ser Phe Ser Tyr Thr Phe His Gly
   85 90 95
   Asp Pro His Ala Met Phe Ala Glu Phe Phe Gly Gly Arg Asn Pro Phe
   100 105 110
   Asp Thr Phe Phe Gly Gln Arg Asn Gly Glu Glu Gly Met Asp Ile Asp
   115 120 125
   Asp Pro Phe Ser Gly Phe Pro Met Gly Met Gly Gly Phe Thr Asn Val
   130 135 140
   Asn Phe Gly Arg Ser Arg Ser Ala Gln Glu Pro Ala Arg Lys Lys Gln
   145 150 155 160
   Asp Pro Pro Val Thr His Asp Leu Arg Val Ser Leu Glu Glu Ile Tyr
   165 170 175
   Ser Gly Cys Thr Lys Lys Met Lys Ile Ser His Lys Arg Leu Asn Pro
   180 185 190
   Asp Gly Lys Ser Ile Arg Asn Glu Asp Lys Ile Leu Thr Ile Glu Val
   195 200 205
   Lys Lys Gly Trp Lys Glu Gly Thr Lys Ile Thr Phe Pro Lys Glu Gly
   210 215 220
   Asp Gln Thr Ser Asn Asn Ile Pro Ala Asp Ile Val Phe Val Leu Lys
   225 230 235 240
   Asp Lys Pro His Asn Ile Phe Lys Arg Asp Gly Ser Asp Val Ile Tyr
   245 250 255
   Pro Ala Arg Ile Ser Leu Arg Glu Ala Leu Cys Gly Cys Thr Val Asn
   260 265 270
   Val Pro Thr Leu Asp Gly Arg Thr Ile Pro Val Val Phe Lys Asp Val
   275 280 285
   Ile Arg Pro Gly Met Arg Arg Lys Val Pro Gly Glu Gly Leu Pro Leu
   290 295 300
   Pro Lys Thr Pro Glu Lys Arg Gly Asp Leu Ile Ile Glu Phe Glu Val
   305 310 315 320
   Ile Phe Pro Glu Arg Ile Pro Gln Thr Ser Arg Thr Val Leu Glu Gln
   325 330 335
   Val Leu Pro Ile
   340
<210> 8
   <211> 782
   <212> PRT
   <213> Homo sapiens
<400> 8
<210> 9
   <211> 432
   <212> PRT
   <213> Homo sapiens
<400> 9
<210> 10
   <211> 431
   <212> PRT
   <213> Homo sapiens
<400> 10
<210> 11
   <211> 438
   <212> PRT
   <213> Homo sapiens
<400> 11
   Met Glu Arg Arg Arg Ile Thr Ser Ala Ala Arg Arg Ser Tyr Val Ser
   1 5 10 15
   Ser Gly Glu Met Met Val Gly Gly Leu Ala Pro Gly Arg Arg Leu Gly
   20 25 30
   Pro Gly Thr Arg Leu Ser Leu Ala Arg Met Pro Pro Pro Leu Pro Thr
   35 40 45
   Arg Val Asp Phe Ser Leu Ala Gly Ala Leu Asn Ala Gly Phe Lys Glu
   50 55 60
   Thr Arg Ala Ser Glu Arg Ala Glu Met Met Glu Leu Asn Asp Arg Phe
   65 70 75 80
   Ala Ser Tyr Ile Glu Lys Val Arg Phe Leu Glu Gln Gln Asn Lys Ala
   85 90 95
   Leu Ala Ala Glu Leu Asn Gln Leu Arg Ala Lys Glu Pro Thr Lys Leu
   100 105 110
   Ala Asp Val Tyr Gln Ala Glu Leu Arg Glu Leu Arg Leu Arg Leu Asp
   115 120 125
   Gln Leu Thr Ala Asn Ser Ala Arg Leu Glu Val Glu Arg Asp Asn Leu
   130 135 140
   Ala Gln Asp Leu Ala Thr Val Arg Gln Lys Leu Gln Asp Glu Thr Asn
   145 150 155 160
   Leu Arg Leu Glu Ala Glu Asn Asn Leu Ala Ala Tyr Arg Gln Glu Ala
   165 170 175
   Asp Glu Ala Thr Leu Ala Arg Leu Asp Leu Glu Arg Lys Ile Glu Ser
   180 185 190
   Leu Glu Glu Glu Ile Arg Phe Leu Arg Lys Ile His Glu Glu Glu Val
   195 200 205
   Arg Glu Leu Gln Glu Gln Leu Ala Arg Gln Gln Val His Val Glu Leu
   210 215 220
   Asp Val Ala Lys Pro Asp Leu Thr Ala Ala Leu Lys Glu Ile Arg Thr
   225 230 235 240
   Gln Tyr Glu Ala Met Ala Ser Ser Asn Met His Glu Ala Glu Glu Trp
   245 250 255
   Tyr Arg Ser Lys Phe Ala Asp Leu Thr Asp Ala Ala Ala Arg Asn Ala
   260 265 270
   Glu Leu Leu Arg Gln Ala Lys His Glu Ala Asn Asp Tyr Arg Arg Gln
   275 280 285
   Leu Gln Ser Leu Thr Cys Asp Leu Glu Ser Leu Arg Gly Thr Asn Glu
   290 295 300
   Ser Leu Glu Arg Gln Met Arg Glu Gln Glu Glu Arg His Val Arg Glu
   305 310 315 320
   Ala Ala Ser Tyr Gln Glu Ala Leu Ala Arg Leu Glu Glu Glu Gly Gln
   325 330 335
   Ser Leu Lys Asp Glu Met Ala Arg His Leu Gln Glu Tyr Gln Asp Leu
   340 345 350
   Leu Asn Val Lys Leu Ala Leu Asp Ile Glu Ile Ala Thr Tyr Arg Lys
   355 360 365
   Leu Leu Glu Gly Glu Glu Asn Arg Ile Thr Ile Pro Val Gln Thr Phe
   370 375 380
   Ser Asn Leu Gln Ile Arg Gly Gln Tyr Ser Arg Ala Ser Trp Glu Gly
   385 390 395 400
   His Trp Ser Pro Ala Pro Ser Ser Arg Ala Cys Arg Leu Leu Gln Thr
                   405                 410                 415
   Gly Thr Glu Asp Gln Gly Lys Gly Ile Gln Leu Ser Leu Gly Ala Phe
               420                 425                 430
   Val Thr Leu Gln Arg Ser
           435
<210> 12
   <211> 335
   <212> PRT
   <213> Homo sapiens
<400> 12
   Met Gly Lys Val Lys Val Gly Val Asn Gly Phe Gly Arg Ile Gly Arg
   1               5                   10                  15
   Leu Val Thr Arg Ala Ala Phe Asn Ser Gly Lys Val Asp Ile Val Ala
               20                  25                  30
   Ile Asn Asp Pro Phe Ile Asp Leu Asn Tyr Met Val Tyr Met Phe Gln
           35                  40                  45
   Tyr Asp Ser Thr His Gly Lys Phe His Gly Thr Val Lys Ala Glu Asn
       50                  55                  60
   Gly Lys Leu Val Ile Asn Gly Asn Pro Ile Thr Ile Phe Gln Glu Arg
   65                  70                  75                  80
   Asp Pro Ser Lys Ile Lys Trp Gly Asp Ala Gly Ala Glu Tyr Val Val
                   85                  90                  95
   Glu Ser Thr Gly Val Phe Thr Thr Met Glu Lys Ala Gly Ala His Leu
               100                 105                 110
   Gln Gly Gly Ala Lys Arg Val Ile Ile Ser Ala Pro Ser Ala Asp Ala
           115                 120                 125
   Pro Met Phe Val Met Gly Val Asn His Glu Lys Tyr Asp Asn Ser Leu
       130                 135                 140
   Lys Ile Ile Ser Asn Ala Ser Cys Thr Thr Asn Cys Leu Ala Pro Leu
   145                 150                 155                 160
   Ala Lys Val Ile His Asp Asn Phe Gly Ile Val Glu Gly Leu Met Thr
                   165                 170                 175
   Thr Val His Ala Ile Thr Ala Thr Gln Lys Thr Val Asp Gly Pro Ser
               180                 185                 190
   Gly Lys Leu Trp Arg Asp Gly Arg Gly Ala Leu Gln Asn Ile Ile Pro
   195 200 205
   Ala Ser Thr Gly Ala Ala Lys Ala Val Gly Lys Val Ile Pro Glu Leu
   210 215 220
   Asn Gly Lys Leu Thr Gly Met Ala Phe Arg Val Pro Thr Ala Asn Val
   225 230 235 240
   Ser Val Val Asp Leu Thr Cys Arg Leu Glu Lys Pro Ala Lys Tyr Asp
   245 250 255
   Asp Ile Lys Lys Val Val Lys Gln Ala Ser Glu Gly Pro Leu Lys Gly
   260 265 270
   Ile Leu Gly Tyr Thr Glu His Gln Val Val Ser Ser Asp Phe Asn Ser
   275 280 285
   Asp Thr His Ser Ser Thr Phe Asp Ala Gly Ala Gly Ile Ala Leu Asn
   290 295 300
   Asp His Phe Val Lys Leu Ile Ser Trp Tyr Asp Asn Glu Phe Gly Tyr
   305 310 315 320
   Ser Asn Arg Val Val Asp Leu Met Ala His Met Ala Ser Lys Glu
   325 330 335
<210> 13
   <211> 353
   <212> PRT
   <213> Homo sapiens
<400> 13
<210> 14
   <211> 162
   <212> PRT
   <213> Homo sapiens
<400> 14
   Met Leu Phe Gly Arg Ile Pro Leu Arg Tyr Ala Ile Leu Met Gln Met
   1               5                   10                  15
   Arg Phe Asp Gly Arg Leu Gly Phe Pro Gly Gly Phe Val Asp Thr Gln
               20                  25                  30
   Asp Arg Ser Leu Glu Asp Gly Leu Asn Arg Glu Leu Arg Glu Glu Leu
           35                  40                  45
   Gly Glu Ala Ala Ala Ala Phe Arg Val Glu Arg Thr Asp Tyr Arg Ser
       50                  55                  60
   Ser His Val Gly Ser Gly Pro Arg Val Val Ala His Phe Tyr Ala Lys
   65                  70                  75                  80
   Arg Leu Thr Leu Glu Glu Leu Leu Ala Val Glu Ala Gly Ala Thr Arg
                   85                  90                  95
   Ala Lys Asp His Gly Leu Glu Val Leu Gly Leu Val Arg Val Pro Leu
               100                 105                 110
   Tyr Thr Leu Arg Asp Gly Val Gly Gly Leu Pro Thr Phe Leu Glu Asn
           115                 120                 125
   Ser Phe Ile Gly Ser Ala Arg Glu Gln Leu Leu Glu Ala Leu Gln Asp
       130                 135                 140
   Leu Gly Leu Leu Gln Ser Gly Ser Ile Ser Gly Leu Lys Ile Pro Ala
   145                 150                 155                 160
   His His
<210> 15
   <211> 97
   <212> PRT
   <213> Homo sapiens
<400> 15
   Met Ser Ser Gly Asn Ala Lys Ile Gly His Pro Ala Pro Asn Phe Lys
   1               5                   10                  15
   Ala Thr Ala Asp Glu Gly Ile Ser Phe Arg Gly Leu Phe Ile Ile Asp
               20                  25                  30
   Asp Lys Gly Ile Leu Arg Gln Ile Thr Val Asn Asp Leu Pro Val Gly
           35                  40                  45
   Arg Ser Val Asp Glu Thr Leu Arg Leu Val Gln Ala Phe Gln Phe Thr
   50 55 60
   Asp Lys His Gly Glu Val Cys Pro Ala Gly Trp Lys Pro Gly Ser Asp
   65 70 75 80
   Thr Ile Lys Pro Asp Val Gln Lys Ser Lys Glu Tyr Phe Ser Lys Gln
   85 90 95
   Lys
<210> 16
   <211> 171
   <212> PRT
   <213> Homo sapiens
<400> 16
   Met Ser Ser Gly Asn Ala Lys Ile Gly His Pro Ala Pro Asn Phe Lys
   1 5 10 15
   Ala Thr Ala Val Met Pro Asp Gly Gln Phe Lys Asp Ile Ser Leu Ser
   20 25 30
   Asp Tyr Lys Gly Lys Tyr Val Val Phe Phe Phe Tyr Pro Leu Asp Phe
   35 40 45
   Thr Phe Val Cys Pro Thr Glu Ile Ile Ala Phe Ser Asp Arg Ala Glu
   50 55 60
   Glu Phe Lys Lys Leu Asn Cys Gln Val Ile Gly Ala Ser Val Asp Ser
   65 70 75 80
   His Phe Cys His Leu Ala Trp Val Asn Thr Pro Lys Lys Gln Gly Gly
   85 90 95
   Leu Gly Pro Met Asn Ile Pro Leu Val Ser Asp Pro Lys Arg Thr Ile
   100 105 110
   Ala Gln Asp Tyr Gly Val Leu Lys Ala Asp Glu Gly Ile Ser Phe Arg
   115 120 125
   Gly Leu Phe Ile Ile Asp Asp Lys Gly Ile Leu Arg Gln Ile Thr Val
   130 135 140
   Asn Asp Leu Pro Val Gly Arg Ser Val Asp Glu Thr Leu Arg Leu Val
   145 150 155 160
   Gln Ala Phe Gln Phe Thr Asp Lys His Gly Glu
   165 170
<210> 17
   <211> 224
   <212> PRT
   <213> Homo sapiens
<400> 17
   Met Pro Gly Gly Leu Leu Leu Gly Asp Val Ala Pro Asn Phe Glu Ala
   1 5 10 15
   Asn Thr Thr Val Gly Arg Ile Arg Phe His Asp Phe Leu Gly Asp Ser
   20 25 30
   Trp Gly Ile Leu Phe Ser His Pro Arg Asp Phe Thr Pro Val Cys Thr
   35 40 45
   Thr Glu Leu Gly Arg Ala Ala Lys Leu Ala Pro Glu Phe Ala Lys Arg
   50 55 60
   Asn Val Lys Leu Ile Ala Leu Ser Ile Asp Ser Val Glu Asp His Leu
   65 70 75 80
   Ala Trp Ser Lys Asp Ile Asn Ala Tyr Asn Cys Glu Glu Pro Thr Glu
   85 90 95
   Lys Leu Pro Phe Pro Ile Ile Asp Asp Arg Asn Arg Glu Leu Ala Ile
   100 105 110
   Leu Leu Gly Met Leu Asp Pro Ala Glu Lys Asp Glu Lys Gly Met Pro
   115 120 125
   Val Thr Ala Arg Val Val Phe Val Phe Gly Pro Asp Lys Lys Leu Lys
   130 135 140
   Leu Ser Ile Leu Tyr Pro Ala Thr Thr Gly Arg Asn Phe Asp Glu Ile
   145 150 155 160
   Leu Arg Val Val Ile Ser Leu Gln Leu Thr Ala Glu Lys Arg Val Ala
   165 170 175
   Thr Pro Val Asp Trp Lys Asp Gly Asp Ser Val Met Val Leu Pro Thr
   180 185 190
   Ile Pro Glu Glu Glu Ala Lys Lys Leu Phe Pro Lys Gly Val Phe Thr
   195 200 205
   Lys Glu Leu Pro Ser Gly Lys Lys Tyr Leu Arg Tyr Thr Pro Gln Pro
   210 215 220
<210> 18
   <211> 357
   <212> PRT
   <213> Homo sapiens
<400> 18
<210> 19
   <211> 585
   <212> PRT
   <213> Homo sapiens
<400> 19
   Pro Gln Pro Ile Asn Val Arg Val Thr Thr Met Asp Ala Glu Leu Glu
   1 5 10 15
   Phe Ala Ile Gln Pro Asn Thr Thr Gly Lys Gln Leu Phe Asp Gln Val
   20 25 30
   Val Lys Thr Ile Gly Leu Arg Glu Val Trp Tyr Phe Gly Leu His Tyr
   35 40 45
   Val Asp Asn Lys Gly Phe Pro Thr Trp Leu Lys Leu Asp Lys Lys Val
   50 55 60
   Ser Ala Gln Glu Val Arg Lys Glu Asn Pro Leu Gln Phe Lys Phe Arg
   65 70 75 80
   Ala Lys Phe Tyr Pro Glu Asp Val Ala Glu Glu Leu Ile Gln Asp Ile
   85 90 95
   Thr Gln Lys Leu Phe Phe Leu Gln Val Lys Glu Gly Ile Leu Ser Asp
   100 105 110
   Glu Ile Tyr Cys Pro Pro Glu Thr Ala Val Leu Leu Gly Ser Tyr Ala
   115 120 125
   Val Gln Ala Lys Phe Gly Asp Tyr Asn Lys Glu Val His Lys Ser Gly
   130 135 140
   Tyr Leu Ser Ser Glu Arg Leu Ile Pro Gln Arg Val Met Asp Gln His
   145 150 155 160
   Lys Leu Thr Arg Asp Gln Trp Glu Asp Arg Ile Gln Val Trp His Ala
   165 170 175
   Glu His Arg Gly Met Leu Lys Asp Asn Ala Met Leu Glu Tyr Leu Lys
   180 185 190
   Ile Ala Gln Asp Leu Glu Met Tyr Gly Ile Asn Tyr Phe Glu Ile Lys
   195 200 205
   Asn Lys Lys Gly Thr Asp Leu Trp Leu Gly Val Asp Ala Leu Gly Leu
   210 215 220
   Asn Ile Tyr Glu Lys Asp Asp Lys Leu Thr Pro Lys Ile Gly Phe Pro
   225 230 235 240
   Trp Ser Glu Ile Arg Asn Ile Ser Phe Asn Asp Lys Lys Phe Val Ile
   245 250 255
   Lys Pro Ile Asp Lys Lys Ala Pro Asp Phe Val Phe Tyr Ala Pro Arg
   260 265 270
   Leu Arg Ile Asn Lys Arg Ile Leu Gln Leu Cys Met Gly Asn His Glu
   275 280 285
   Leu Tyr Met Arg Arg Arg Lys Pro Asp Thr Ile Glu Val Gln Gln Met
   290 295 300
   Lys Ala Gln Ala Arg Glu Glu Lys His Gln Lys Gln Leu Glu Arg Gln
   305 310 315 320
   Gln Leu Glu Thr Glu Lys Lys Arg Arg Glu Thr Val Glu Arg Glu Lys
   325 330 335
   Glu Gln Met Met Arg Glu Lys Glu Glu Leu Met Leu Arg Leu Gln Asp
   340 345 350
   Tyr Glu Glu Lys Thr Lys Lys Ala Glu Arg Glu Leu Ser Glu Gln Ile
   355 360 365
   Gln Arg Ala Leu Gln Leu Glu Glu Glu Arg Lys Arg Ala Gln Glu Glu
   370 375 380
   Ala Glu Arg Leu Glu Ala Asp Arg Met Ala Ala Leu Arg Ala Lys Glu
   385 390 395 400
   Glu Leu Glu Arg Gln Ala Val Asp Gln Ile Lys Ser Gln Glu Gln Leu
   405 410 415
   Ala Ala Glu Leu Ala Glu Tyr Thr Ala Lys Ile Ala Leu Leu Glu Glu
   420 425 430
   Ala Arg Arg Arg Lys Glu Asp Glu Val Glu Glu Trp Gln His Arg Ala
   435 440 445
   Lys Glu Ala Gln Asp Asp Leu Val Lys Thr Lys Glu Glu Leu His Leu
   450 455 460
   Val Met Thr Ala Pro Pro Pro Pro Pro Pro Pro Val Tyr Glu Pro Val
   465 470 475 480
   Ser Tyr His Val Gln Glu Ser Leu Gln Asp Glu Gly Ala Glu Pro Thr
   485 490 495
   Gly Tyr Ser Ala Glu Leu Ser Ser Glu Gly Ile Arg Asp Asp Arg Asn
   500 505 510
   Glu Glu Lys Arg Ile Thr Glu Ala Glu Lys Asn Glu Arg Val Gln Arg
   515 520 525
   Gln Leu Leu Thr Leu Ser Ser Glu Leu Ser Gln Ala Arg Asp Glu Asn
   530 535 540
   Lys Arg Thr His Asn Asp Ile Ile His Asn Glu Asn Met Arg Gln Gly
   545 550 555 560
   Arg Asp Lys Tyr Lys Thr Leu Arg Gln Ile Arg Gln Gly Asn Thr Lys
   565 570 575
   Gln Arg Ile Asp Glu Phe Glu Ala Leu
   580 585
<210> 20
   <211> 466
   <212> PRT
   <213> Homo sapiens
<400> 20
   Met Ser Thr Arg Ser Val Ser Ser Ser Ser Tyr Arg Arg Met Phe Gly
   1 5 10 15
   Gly Pro Gly Thr Ala Ser Arg Pro Ser Ser Ser Arg Ser Tyr Val Thr
   20 25 30
   Thr Ser Thr Arg Thr Tyr Ser Leu Gly Ser Ala Leu Arg Pro Ser Thr
   35 40 45
   Ser Arg Ser Leu Tyr Ala Ser Ser Pro Gly Gly Val Tyr Ala Thr Arg
   50 55 60
   Ser Ser Ala Val Arg Leu Arg Ser Ser Val Pro Gly Val Arg Leu Leu
   65 70 75 80
   Gln Asp Ser Val Asp Phe Ser Leu Ala Asp Ala Ile Asn Thr Glu Phe
   85 90 95
   Lys Asn Thr Arg Thr Asn Glu Lys Val Glu Leu Gln Glu Leu Asn Asp
   100 105 110
   Arg Phe Ala Asn Tyr Ile Asp Lys Val Arg Phe Leu Glu Gln Gln Asn
   115 120 125
   Lys Ile Leu Leu Ala Glu Leu Glu Gln Leu Lys Gly Gln Gly Lys Ser
   130 135 140
   Arg Leu Gly Asp Leu Tyr Glu Glu Glu Met Arg Glu Leu Arg Arg Gln
   145 150 155 160
   Val Asp Gln Leu Thr Asn Asp Lys Ala Arg Val Glu Val Glu Arg Asp
   165 170 175
   Asn Leu Ala Glu Asp Ile Met Arg Leu Arg Glu Lys Leu Gln Glu Glu
   180 185 190
   Met Leu Gln Arg Glu Glu Ala Glu Asn Thr Leu Gln Ser Phe Arg Gln
   195 200 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
   450 455 460
   Leu Glu
   465
<210> 22
   <211> 214
   <212> PRT
   <213> Homo sapiens
<400> 22
   Met Gly Leu Ala Gly Val Cys Ala Leu Arg Arg Ser Ala Gly Tyr Ile
   1               5                   10                  15
   Leu Val Gly Gly Ala Gly Gly Gln Ser Ala Ala Ala Ala Ala Arg Arg
               20                  25                  30
   Cys Ser Glu Gly Glu Trp Ala Ser Gly Gly Val Arg Ser Phe Ser Arg
           35                  40                  45
   Ala Ala Ala Ala Met Ala Pro Ile Lys Val Gly Asp Ala Ile Pro Ala
       50                  55                  60
   Val Glu Val Phe Glu Gly Glu Pro Gly Asn Lys Val Asn Leu Ala Glu
   65                  70                  75                  80
   Leu Phe Lys Gly Lys Lys Gly Val Leu Phe Gly Val Pro Gly Ala Phe
                   85                  90                  95
   Thr Pro Gly Cys Ser Lys Thr His Leu Pro Gly Phe Val Glu Gln Ala
               100                 105                 110
   Glu Ala Leu Lys Ala Lys Gly Val Gln Val Val Ala Cys Leu Ser Val
           115                 120                 125
   Asn Asp Ala Phe Val Thr Gly Glu Trp Gly Arg Ala His Lys Ala Glu
       130                 135                 140
   Gly Lys Val Arg Leu Leu Ala Asp Pro Thr Gly Ala Phe Gly Lys Glu
   145                 150                 155                 160
   Thr Asp Leu Leu Leu Asp Asp Ser Leu Val Ser Ile Phe Gly Asn Arg
                   165                 170                 175
   Arg Leu Lys Arg Phe Ser Met Val Val Gln Asp Gly Ile Val Lys Ala
               180                 185                 190
   Leu Asn Val Glu Pro Asp Gly Thr Gly Leu Thr Cys Ser Leu Ala Pro
           195                 200                 205
   Asn Ile Ile Ser Gln Leu
       210
<210> 23
   <211> 410
   <212> PRT
   <213> Homo sapiens
<400> 23
<210> 24
   <211> 594
   <212> PRT
   <213> Homo sapiens
<400> 24
   Met Ala Pro Ile Gly Leu Lys Ala Val Val Gly Glu Lys Ile Met His
   1 5 10 15
   Asp Val Ile Lys Lys Val Lys Lys Lys Gly Glu Trp Lys Val Leu Val
               20                  25                  30
   Val Asp Gln Leu Ser Met Arg Met Leu Ser Ser Cys Cys Lys Met Thr
           35                  40                  45
   Asp Ile Met Thr Glu Gly Ile Thr Ile Val Glu Asp Ile Asn Lys Arg
       50                  55                  60
   Arg Glu Pro Leu Pro Ser Leu Glu Ala Val Tyr Leu Ile Thr Pro Ser
   65                  70                  75                  80
   Glu Lys Ser Val His Ser Leu Ile Ser Asp Phe Lys Asp Pro Pro Thr
                   85                  90                  95
   Ala Lys Tyr Arg Ala Ala His Val Phe Phe Thr Asp Ser Cys Pro Asp
               100                 105                 110
   Ala Leu Phe Asn Glu Leu Val Lys Ser Arg Ala Ala Lys Val Ile Lys
           115                 120                 125
   Thr Leu Thr Glu Ile Asn Ile Ala Phe Leu Pro Tyr Glu Ser Gln Val
       130                 135                 140
   Tyr Ser Leu Asp Ser Ala Asp Ser Phe Gln Ser Phe Tyr Ser Pro His
   145                 150                 155                 160
   Lys Ala Gln Met Lys Asn Pro Ile Leu Glu Arg Leu Ala Glu Gln Ile
                   165                 170                 175
   Ala Thr Leu Cys Ala Thr Leu Lys Glu Tyr Pro Ala Val Arg Tyr Arg
               180                 185                 190
   Gly Glu Tyr Lys Asp Asn Ala Leu Leu Ala Gln Leu Ile Gln Asp Lys
           195                 200                 205
   Leu Asp Ala Tyr Lys Ala Asp Asp Pro Thr Met Gly Glu Gly Pro Asp
       210                 215                 220
   Lys Ala Arg Ser Gln Leu Leu Ile Leu Asp Arg Gly Phe Asp Pro Ser
   225                 230                 235                 240
   Ser Pro Val Leu His Glu Leu Thr Phe Gln Ala Met Ser Tyr Asp Leu
                   245                 250                 255
   Leu Pro Ile Glu Asn Asp Val Tyr Lys Tyr Glu Thr Ser Gly Ile Gly
               260                 265                 270
   Glu Ala Arg Val Lys Glu Val Leu Leu Asp Glu Asp Asp Asp Leu Trp
           275                 280                 285
   Ile Ala Leu Arg His Lys His Ile Ala Glu Val Ser Gln Glu Val Thr
   290 295 300
   Arg Ser Leu Lys Asp Phe Ser Ser Ser Lys Arg Met Asn Thr Gly Glu
   305 310 315 320
   Lys Thr Thr Met Arg Asp Leu Ser Gln Met Leu Lys Lys Met Pro Gln
   325 330 335
   Tyr Gln Lys Glu Leu Ser Lys Tyr Ser Thr His Leu His Leu Ala Glu
   340 345 350
   Asp Cys Met Lys His Tyr Gln Gly Thr Val Asp Lys Leu Cys Arg Val
   355 360 365
   Glu Gln Asp Leu Ala Met Gly Thr Asp Ala Glu Gly Glu Lys Ile Lys
   370 375 380
   Asp Pro Met Arg Ala Ile Val Pro Ile Leu Leu Asp Ala Asn Val Ser
   385 390 395 400
   Thr Tyr Asp Lys Ile Arg Ile Ile Leu Leu Tyr Ile Phe Leu Lys Asn
   405 410 415
   Gly Ile Thr Glu Glu Asn Leu Asn Lys Leu Ile Gln His Ala Gln Ile
   420 425 430
   Pro Pro Glu Asp Ser Glu Ile Ile Thr Asn Met Ala His Leu Gly Val
   435 440 445
   Pro Ile Val Thr Asp Ser Thr Leu Arg Arg Arg Ser Lys Pro Glu Arg
   450 455 460
   Lys Glu Arg Ile Ser Glu Gln Thr Tyr Gln Leu Ser Arg Trp Thr Pro
   465 470 475 480
   Ile Ile Lys Asp Ile Met Glu Asp Thr Ile Glu Asp Lys Leu Asp Thr
   485 490 495
   Lys His Tyr Pro Tyr Ile Ser Thr Arg Ser Ser Ala Ser Phe Ser Thr
   500 505 510
   Thr Ala Val Ser Ala Arg Tyr Gly His Trp His Lys Asn Lys Ala Pro
   515 520 525
   Gly Glu Tyr Arg Ser Gly Pro Arg Leu Ile Ile Phe Ile Leu Gly Gly
   530 535 540
   Val Ser Leu Asn Glu Met Arg Cys Ala Tyr Glu Val Thr Gln Ala Asn
   545 550 555 560
   Gly Lys Trp Glu Val Leu Ile Gly Ser Thr His Ile Leu Thr Pro Gln
                   565                 570                 575
   Lys Leu Leu Asp Thr Leu Lys Lys Leu Asn Lys Thr Asp Glu Glu Ile
               580                 585                 590
   Ser Ser

## Beschreibung

Die Erfindung betrifft ein Verfahren zur Diagnose und Risikostratifizierung von Hirntumoren, insbesondere Gliome, Meningeome und Hypophysenadenome, wobei eine Bestimmung mittels ausgewählten Biomarkern erfolgt. Ferner betrifft die Erfindung hierzu geeignete Kombinationen von Biomarkern, insbesondere zur in-vitro Diagnostik.

Primäre Hirntumore, wie Gliome, Meningeome und Hypophysenadenome, gehen von Neuroepithel, Ganglienzellen, Meningen, Nervenscheiden, allgemeinem Nervenstützgewebe bzw. Neuroglia und Hypophyse oder ektopen intrakraniellen Geweben (Keimzell- od. Fehlbildungstumoren) aus und deren Ursache sind vor allem in genetischen und hormonalen Faktoren, onkogene Viren und exogene Karzinogene zu sehen. Die WHO-Gradeinteilung erfolgt entsprechend der Dignität: Grad I (gutartig / benigne), Grad II (semibenigne; postoperative Überlebenszeit 3-5 Jahre), Grad III (semimaligne; postoperative Überlebenszeit 2-3 Jahre), Grad IV (maligne; postoperative Überlebenszeit 6-15 Mon.); Häufigkeit: Anteil der gesamten bzw. primären Hirntumoren an allen Tumorerkrankungen: 7-9 % (Kleihues, P., Louis, D. N., Scheithauer, B. W., Rorke, L. B., Reifenberger, G., Burger, P. C., and Cavenee, W. K. (2002) The WHO classification of tumors of the nervous system. J. Neuropathol. Exp. Neurol. 3, 215-225).

Ein Gliom wird histologisch in (Riesenzell-) (Oligo-) Astrozytomen, Oligodendrogliom, Mischgliome, Glioblastom multiforme charakterisiert und differenziert je nach Wachstum, wie isomorph, anaplastisch, pilozystisch u.a.. Ferner können Untergruppen von Gliomen anhand des Verlustes der Heterozygotie (loss of heterocygosity (im Folgenden LOH)) prognostiziert werden, z.B. zu den humanen Chromosomen(-Loci) 1p und 19q (Reifenberger, G., and Louis, D. N. (2003) Oligodendroglioma: toward molecular definitions in diagnostic neuro-oncology. J. Neuropathol. Exp. Neurol., 62, 111-126, Louis, D. N. (2006) Molecular Pathology of Malignant Gliomas. Annu. Rev. Path. Mech. Dis. 1, 97-117, Smith, J. S., and Jenkins, R. B. (2000) Genetic "alterations in adult diffuse glioma: occurrence, significance, and prognostic implications. Front Biosci. 5, 213-231), die zum Verlust von Tumorsuppressorgenen führen (Felsberg, J., Erkwoh, A., Sabel, M., Kirsch, L., Fimmers, R., Blaschke, B., Schlegel, U., Schramm, J., Wiestler, O. D., and Reifenberger, G. (2004) Allelic losses on 1 p and 19q in oligodendroglial tumors: Refinement of candidate tumor suppressor regions on 1p and correlation with survival data. Brain Pathol 14, 121-130, Tews, B., Felsberg, J., Hartmann, C., Kunitz, A., Hahn, M., Toedt, G., Neben, K., Hummerich, L., von Deimling, A., Reifenberger, G., and Lichter, P. (2006) Identification of novel oligodendroglioma-associated candidate tumor suppressor genes in 1p36 and 19q13 using microarray-based expression profiling. Int J Cancer. 119, 792-800).

Allerdings ist die Identifizierung von Tumorsuppressorgenen für die Diagnose und Risikostratifizierung von Patienten nicht ausreichend und es besteht ein Bedarf zur Identifizierung von Biomarkern zur geeigneten Diagnose und Risikostratifizierung von Patienten.

WO2004/016758 und offenbaren Proteine zur Diagnose von Gliomen, solche wie Enzyme, Wachstumsfaktoren u.a.

WO2002/0822076 offenbart die Eignung von Aldose reductase zur Diagnose von Krebs samt einem zugehörigen Kit.

Zwecks einer geeigneten Therapie von Hirntumoren, insbesondere von Gliomen, bedarf es einer frühen Diagnose und Differenzierung in Verbindung mit der Notwendigkeit klinische Entscheidungen zu treffen.

Eine der Erfindung zugrunde liegende Aufgabe besteht daher darin, ein Verfahren zur Diagnose von Hirntumoren, insbesondere Gliomen zu entwickeln, das eine verbesserte frühzeitige Diagnose sowie verbesserte Erfassung von Risikopatienten ermöglicht und den Therapieerfolg verbessert.

Die Aufgabe wird durch ein Verfahren zur Diagnose und / oder Risikostratifizierung von Hirntumoren, insbesondere Gliomen gelöst, wobei eine Bestimmung von Aldose reductase (SEQ ID No. 1, Acc. No. IPI00413641.6) von einem zu untersuchenden Patienten erfolgt, oder eine Bestimmung von Aldose reductase (SEQ ID No. 1, Acc. No. IPI00413641.6) in Kombination mit mindestens einem Biomarker ausgewählt aus der Gruppe Alpha crystallin B chain (SEQ ID No. 2, Acc. No. IPI00021369.1),
Alpha-enolase (SEQ ID No. 3, Acc. No. IPI00465248.4),
Annexin A1, kurz ANXA1 (SEQ ID No. 4, Acc. No. IPI00218918.4), Annexin A5 (SEQ ID No. 5, Acc. No. IPI00329801.11),
Chloride Intracellular channel protein 1, kurz CLIC1 (SEQ ID No. 6, Acc. No. IPI00010896.2),
DnaJ homolog subfamily B member 1 (SEQ ID No. 7, Acc. No. IPI00015947.4),
Gelsolin precursor (SEQ ID No. 8, Acc. No. IPI00026314.1),
Glial fibrillary acidic protein (SEQ ID No. 9, Acc. No. IPI00025362.1),
Glial fibrillary acidic protein (SEQ ID No. 10, Acc. No. IPI00443478.1),
Glial fibrillary acidic protein (SEQ ID No. 11, Acc. No. IPI00383815.4),
Glyceraldehyde-3-phosphate-dehydrogenase (SEQ ID No. 12, Acc. No. IPI00219018.6),
Heterogenous nuclear ribonucleoprotein (SEQ ID No. 13, Acc. No. IPI00477522.1),
Nucleosidediphosphate-linked moiety X motif 16 (SEQ ID No. 14, Acc. No. IPI00411732.4),
Peroxiredoxin-1, kurz PRDX1 (SEQ ID No. 15, Acc. No. IPI00641244.1),
Peroxiredoxin-1, kurz PRDX1 (SEQ ID No. 16, Acc. No. IPI00640741.1),
Peroxiredoxin-6 (SEQ ID No. 17, Acc. No. IPI00220301.4),
Sorbitoldehydrogenase (SEQ ID No. 18, Acc. No. IPI00216057.4),
Villin-2, kurz VIL2 (SEQ ID No. 19, Acc. No. IPI00479359.5),
Vimentin, kurz VIM (SEQ ID No. 20, Acc. No. IPI00418471.5),
Vimentin, kurz VIM (SEQ ID No. 21, Acc. No. IPI00552689.1), und
Peroxiredoxin-5 mitochondrialprecursor (SEQ ID No. 22, Acc. No. IPI00024915.2),
Platelet-activating factor acetylhydrolase IB alpha unit (SEQ ID No. 23, Acc. No. IPI00218728.2),
Syntaxin-binding protein-1 (SEQ ID No. 24, Acc. No. IPI00641244.1),
von einem zu untersuchenden Patienten erfolgt (nachstehend erfindungsgemäßes Verfahren mittels erfindungsgemäßer (Bio) marker).

Die genannten erfindungsgemäßen Proteine sind in Abhängigkeit des LOH-Status anhand der humanen Chromosomen (-Loci) 1p und 19q charakteristisch hoch- oder runter-geregelt (exprimiert). Hochgeregelt sind: SEQ ID No. 1 - 21, runtergeregelt: SEQ ID No.22-24.

Die erfindungsgemäßen Proteine konnten mittels einer differentiellen Proteomanalyse von Tumorgewebe identifiziert werden. Hierzu wurden von erkrankten Patienten entsprechende Tumorproben entnommen. Die Proben wurden in einem Handhomogenisator mit Lysispuffer homogenisiert und von DNA und sonstigem Zellmaterial befreit, um ein Proteinkonzentrat zu erhalten. Die Proteine wurden mit einem Farbstoff markiert und einer 2D-Gelelektrophorese unterworfen mit einer isoelektrischen Fokussierung in der ersten und einer SDS-Gelelektrophorese in der zweiten Dimension.

Die differentielle Darstellung (LOH/ohne LOH (=Retention, kurz RET) ist in der Tabelle 1 und Beispielen und Abbildungen dargelegt und zeigen unterschiedliche charakteristische Expression in Abhängigkeit mit dem LOH-Status (hoch- (up-) und runter- (down-) reguliert, ausgelesen anhand der Spots).

**Tabelle 1:**

| N o. | t-Test | FC | IPI | Protein | Chrom osom | pl-Seq | Mr-Seq. | pl-2D | Mr-2D | Pepti de count | Seq. Coverage | Prof ound-Scor e | Mascot-Score |
|---|---|---|---|---|---|---|---|---|---|---|---|---|---|
| **Hochreguliert in Proben mit RET** | | | | | | | | | | | | | |
| 1 | 0,011 | 9,7 | 00383815.4 | Splice Isoform 2 of Glial fibrillary acidic protein, astrocyte | 17q21 | 5,4 | 50,3 | 5,22 | 17,6 | 11(1) | 27,2 | 2,3 | 84,6 |
| 2 | 0,014 | 7,7 | 00025363.1 | Splice Isoform 1Glial fibrillary acidic protein, astrocyte* | 17q21 | 5,4 | 50,3 | 5,03 | 58,9 | 30(0) | 46,9 | 2,3 | 214 |
| 3 | 0,012 | 6,7 | 00418471.5 | Vimentin | 10p13 | 4,9 | 53,5 | 5,14 | 61,9 | 39 | 71,8 | 2,3 | 300 |
| 4 | 0,047 | 6,6 | 00418471.5 | Vimentin | 10p13 | 4,9 | 53,5 | 5,1 | 61,9 | 30 | 61,7 | 2,3 | 229 |
| 5 | 0,017 | 6,4 | 00218918.4 | Annexin A1 | 9q12-q21.2 | 6,7 | 38,6 | 6,68 | 44 | 15 | 56,8 | 2,4 | 204 |
| 6 | 0,01 | 5,7 | 00383815.4 | Splice Isoform 2 of Glial fibrillary acidic protein, astrocyte | 17q21 | 5,4 | 50,3 | 5,07 | 58,8 | 26(1) | 50,7 | 2,2 | 242 |
| 7 | 0,005 9 | 4,8 | 00418471.5 | Vimentin | 10p13 | 4,9 | 53,5 | 5,55 | 63,1 | 18 | 61,9 | 2,4 | 220 |
| 8 | 0,038 | 4,8 | 00418471.5 | Vimentin | 10p13 | 4,9 | 53,5 | 4,94 | 50,7 | 34 | 56,1 | 2,3 | 270 |
| 9 | 0,043 | 4,5 | 00443478.1 | Splice Isoform 3 of Glial fibrillary acidic protein, astrocyte* | 17q21 | 5,4 | 50,3 | 5,76 | 54,9 | 40(0) | 44,1 | 2,4 | 362 |
| 10 | 0,032 | 4,2 | 00220301.4 | Peroxiredoxin-6 | 1 q25.1 | 6 | 24,9 | 6,56 | 26,8 | 21 | 70,4 | 2,4 | 205 |
| 11 | 0,004 1 | 4,1 | 00418471.5 | Vimentin | 10p13 | 4,9 | 53,5 | 5,18 | 61,8 | 26 | 58,3 | 2,4 | 233 |
| 12 | 0,011 | 4 | 00443478.1 | Splice Isoform 3 of Glial fibrillary acidic protein, astrocyte* | 17q21 | 5,4 | 50,3 | 5,19 | 20,3 | 7(0) | 15,8 | 1,8 | 75,4 |
| 13 | 0,004 1 | 3,8 | 00443478.1 | Splice Isoform 3 of Glial fibrillary astrocyte* | 17q21 | 5,4 | 50,3 | 5,19 | 19,7 | 12(0) | 24,4 | 2,2 | 93 |
| 14 | 0,037 | 3,5 | 00220301.4 | Peroxiredoxin-6 | 1q25.1 | 6 | 24,9 | 6,48 | 26,8 | 13 | 68,2 | 2,3 | 172 |
| 15 | 0,049 | 3,4 | 00383815.4 | Splice Isoform 1 of Glial fibrillary acidic protein, astrocyte | 17q21 | 5,4 | 50,3 | 5,11 | 18,3 | 23(1) | 44 | 2,2 | 150 |
| 16 | 0,004 5 | 3,3 | 00216057.4 | Sorbitol dehydrogenase | 15q15. 3 | 9,3 | 38,1 | 7,48 | 46,3 | 15 | 43,8 | 2,2 | 86,3 |
| 17 | 0,018 | 3,3 | 00479359.5 | Villin2 | 6q25.2 -q26 | 5,9 | 69,2 | 6,55 | 90,2 | 35 | 55,7 | 2,4 | 272 |
| 18 | 0,017 | 3,3 | 00552689.1 | Vimentin | 10p13 | 4,7 | 20 | 4,81 | 48,6 | 7 | 32,4 | 1,3 | 86,9 |
| 19 | 0,025 | 3,2 | 00383815.4 | Splice Isoform 2 of Glial fibrillary acidic protein, astrocyte | 17q21 | 5,4 | 50,3 | 4,9 | 50,8 | 15(1) | 33,6 | 2,1 | 130 |
| 20 | 0,015 | 3,2 | 00413641.6 | Aldose reductase | 7q33 | 6,6 | 35,7 | 6,18 | 26,9 | 7 | 18,7 | 2,3 | 72,8 |
| 21 | 0,039 | 3 | 00025363.1 | Splice Isoform 1 of Glial fibrillary acidic protein, astrocyte | 17q21 | 5,4 | 50,3 | 5,08 | 20,8 | 30(1) | 63 | 2,4 | 276 |
| 22 | 0,014 | 3 | 00443478.1 | Splice Isoform 3 of Glial fibrillary acidic protein, astrocyte | 17q21 | 5,4 | 50,3 | 6,49 | 58,7 | 11 (1) | 20,6 | 1,6 | 61,1 |
| 23 | 0,037 | 3 | 00443478.1 | Splice Isoform 3 of Glial fibrillary acidic protein, astrocyte* | 17q21 | 5,4 | 50,3 | 5,16 | 35,1 | 31 (0) | 59,2 | 2,3 | 267 |
| 24 | 0,032 | 2,9 | 00220301.4 | Peroxiredoxin-6 | 1q25.1 | 6 | 24,9 | 6,51 | 90,7 | 12 | 59,6 | 2,4 | 150 |
| 25 | 0,004 1 | 2,7 | 00479359.5 | Villin2 | 6q25.2 -q26 | 5,9 | 69,2 | 6,13 | 60,5 | 32 | 49,1 | 2,4 | 261 |
| 26 | 0,058 | 2,6 | 00383815.4 | Splice Isoform 2 of Glial fibrillary acidic protein, astrocyte* | 17q21 | 5,4 | 50,3 | 5,51 | 66,3 | 9(0) | 17,4 | 2 | 71,8 |
| 27 | 0,045 | 2,5 | 00418471.5 | Vimentin | 10p13 | 4,9 | 53,5 | 6,17 | 28,3 | 20 | 41,9 | 1,7 | 117 |
| 28 | 0,037 | 2,5 | 00010896.2 | Chloride intracellular channel protein 1 | 6p22.1 -p21.2 | 4,9 | 26,8 | 5,4 | 65,7 | 7 | 35 | 1,5 | 90,3 |
| 29 | 0,009 6 | 2,5 | 00418471.5 | Vimentin | 10p13 | 5 | 49,6 | 5,4 | 65,7 | 21 | 49,4 | 1,8 | 168 |
| 30 | 0,034 | 2,5 | 00418471.5 | Vimentin | 10p13 | 5 | 49,6 | 5,47 | 67,4 | 28 | 60,1 | 2,4 | 274 |
| 31 | 0,003 6 | 2,5 | 00329801.1 1 | Annexin A5 | 4q28-q32 | 4,8 | 35,8 | 5,23 | 35,4 | 9 | 29,2 | 2,4 | 114 |
| 32 | 0,03 | 2,4 | 00021369.1 | Alpha crystallin B chain | 11 q22. 3-q23.1 | 6,9 | 20,1 | 7,27 | 21,5 | 9 | 34,9 | 2,3 | 92,1 |
| 33 | 0,033 | 2,3 | 00443478.1 | Splice Isoform 3 of Glial fibrillary acidic protein, astrocyte* | 17q21 | 5,4 | 50,3 | 6,31 | 58,5 | 30(0) | 56,8 | 2,4 | 278 |
| 34 | 0,043 | 2,3 | 00411732.4 | Nucleoside diphosphate-linked moiety X motif 16 | 3q22.1 | 6,4 | 21,3 | 6,84 | 20,5 | 11 | 50,3 | 2,3 | 102 |
| 35 | 0,034 | 2,2 | 00383815.4 | Splice Isoform 2 of Glial fibrillary acidic protein, astrocyte | 17q21 | 5,4 | 50,3 | 5,5 | 43,7 | 20(1) | 41,3 | 2,4 | 194 |
| 36 | 0,035 | 2,1 | 00026314.1 | Isoform 1 of Gelsolin precursor* | 9q33 | 5,9 | 85,6 | 6,15 | 95,5 | 28(0) | 38,6 | 2,1 | 155 |
| 37 | 0,029 | 2 | 00443478.1 | Splice Isoform 3 of Glial fibrillary acidic protein, astrocyte | 17q21 | 5,4 | 50,3 | 6,36 | 58,9 | 33 | 70,8 | 1,6 | 239 |
| 38 | 0,01 | 2 | 00641244.1 | 11 kDa protein | 1p34.1 | 9,6 | 10,7 | 7,12 | 21,7 | 4 | 43,3 | 1,2 | 71,5 |
| 39 | 0,042 | 2 | 00640741.1 | 19 kDa protein | 1p34.1 | 6,5 | 19 | 7,43 | 21,7 | 5 | 28,1 | 1,1 | 73,9 |
| 40 | 0,023 | 2 | 00477522.1 | Splice Isoform A2 of Heterogeneous nuclear ribonucleoproteins A2/B1* | 7p15 | 9,1 | 36 | 7 | 43,4 | 6(0) | 26,7 | 2,2 | 73,9 |
| 41 | 0,012 | 1,8 | 00219018.6 | Glyceraldehyde-3-phosphate dehydrogenase | 12p13 | 9,3 | 35,9 | 7,69 | 44,2 | 7 | 24,6 | 1,9 | 74,3 |
| 42 | 0,000 91 | 1,8 | 00383815.4 | Splice Isoform 2 of Glial fibrillary acidic protein, astrocyte* | 17q21 | 5,4 | 50,3 | 5,25 | 21,2 | 10(0) | 23,3 | 2,3 | 116 |
| 43 | 0,069 | 1,8 | 00025363.1 | Splice Isoform 1 of Glial fibrillary acidic protein, astrocyte | 17q21 | 5,4 | 50,3 | 4,71 | 55,7 | 18(1) | 39,5 | 2,4 | 108 |
| 44 | 0,032 | 1,8 | 00465248.4 | Alpha-enolase | 1p36.3 -p36.2 | 7,7 | 47 | 6,85 | 58,3 | 12 | 33,3 | 2,3 | 107 |
| 45 | 0,000 64 | 1,8 | 00015947.4 | DnaJ homolog subfamily B member 1 | 19p13. 2 | 9,4 | 37,9 | 7,38 | 46,2 | 8 | 24,2 | 2,3 | 78,8 |
| | | | | | | | | | | | | | |

| **Runterregulierte Proben mit RET** | | | | | | | | | | | | | |
|---|---|---|---|---|---|---|---|---|---|---|---|---|---|
| 46 | 0,038 | -1,8 | IPI0021872 8.2 | Platelet-activating factor acetylhydrolase IB alpha subunit | 17p13. 3 | 7,2 | 46,5 | 7,44 | 62,1 | 11 | 30,8 | 2,3 | 106 |
| 47 | 0,003 4 | -1,8 | IPI0002491 5.2 | Peroxiredoxin-5, mitochondrial precursor | 11q13 | 9,9 | 22 | 6,88 | 13,1 | 6(3) | 36,9 | 2,2 | 95,6 |
| 48 | 0,026 | -1,8 | IPI0008482 | Splice Isoform 1 of Syntaxinbindinq protein 1* | 9q34.1 | 6,5 | 67,5 | 6,73 | 69,5 | 11(0) | 18,5 | 2,3 | 106 |

| | | | | | | | | | | | | | |
|---|---|---|---|---|---|---|---|---|---|---|---|---|---|
| FC = Fold Change, Seq.= Sequenz, Mr= rel. Molekulargewicht, pI= isoelektrischer Punkt, RET = Retention | | | | | | | | | | | | | |

Die Auswertung erfolgte mit Hilfe MALDI-TOF/TOF MS (Matrix gestützte Laser Desorption Ionisation - Flugzeit - Massenspektrometrie. Selbstverständlich können hierbei auch andere geeignete massenspektrometrische Verfahren angewandt werden, beispielsweise von LC-ESI-MS(/MS).

Daher betrifft die Erfindung auch solche Aminosäure-Sequenzen (Polypeptide, Proteine), die eine Sequenzidentität oder Homologie von 70% und mehr, vorzugsweise von 80% und mehr, besonders bevorzugt von 90-95% und mehr mit SEQ ID No. 1 bis SEQ ID No. 24 aufweisen. Ebenfalls mit eingeschlossen sind ebenfalls solche analoge Aminosäure-Sequenzen, die aufgrund des Austausches von einer oder mehreren Aminosäure(n) in diesen Sequenzen, dennoch die gewünschte Funktion eines Biomarkers zur Diagnose von Hirntumoren gewährleisten.

In einer weiteren bevorzugten Ausführungsform sind Kombinationen (Unterkombination aus der obigen Gesamtheit aller erfindungsgemäßen Biomarker) der erfindungsgemäßen Biomarker zur Diagnose von Hirntumoren, insbesondere Gliomen vorteilhaft, insbesondere eine Kombination enthaltend Vimentin (VIM), Villin2 (VIL2) und / oder Annexin 1 (ANXA1).

Der Begriff "Hirntumor" umfasst erfindungsgemäß primäre Hirntumore, wie Gliome, Meningeome und Hypophysenadenome, ausgehend von Neuroepithel, Ganglienzellen, Meningen, Nervenscheiden, allgemeinem Nervenstützgewebe bzw. Neuroglia und Hypophyse oder ektopen intrakraniellen Geweben (Keimzellod. Fehlbildungstumoren) und deren Ursache vor allem in genetischen und hormonalen Faktoren, onkogene Viren und exogene Karzinogene zu sehen sind.

Eine bevorzugte erfindungsgemäße Ausführungsform ist jedoch die Diagnose und Risikostratifizierung von Gliomen.

Die Erfindung betrifft ebenfalls die Identifizierung von Patienten mit erhöhtem Risiko oder/und einer ungünstigen Prognose von Hirntumoren, insbesondere Gliomen.

Das erfindungsgemäße Verfahren ermöglicht daher klinische Entscheidungen, die zu einem schnellen Therapieerfolg und zur Vermeidung von Todesfällen führen. Solche klinische Entscheidungen umfassen ebenfalls weiterführende Behandlung mittels Arzneimitteln zur Behandlung oder Therapie von Hirntumoren, insbesondere Gliomen.

Daher betrifft die Erfindung ebenfalls ein Verfahren zur Diagnose von Patienten mit Hirntumoren, insbesondere Gliomen zur Durchführung von klinischen Entscheidungen, wie weiterführende Behandlung und Therapie mittels Arzneimitteln oder einer Chemotherapie.

In einer weiteren bevorzugten Ausführungsform des erfindungsgemäßen Verfahrens erfolgt die Diagnose zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur Diagnose und zwar zur Früh- oder Differentialdiagnose oder Prognose von Hirntumoren, insbesondere Gliomen, wobei eine Bestimmung der erfindungsgemäßen Biomarker an oder von einem zu untersuchenden Patienten durchgeführt wird.

In einer Ausführungsform des erfindungsgemäßen Verfahrens wird dem zu untersuchenden Patienten Gewebeproben (Biopsie) oder Körperflüssigkeit (Blut, Plasma) entnommen, und die Diagnose erfolgt in vitro/ex vivo, d.h. außerhalb des menschlichen oder tierischen Körpers. Aufgrund der Bestimmung der erfindungsgemäßen Marker wird eine hohe Signifikanz für Hirntumoren, insbesondere Gliomen erzielt und anhand der vorhandenen Menge oder deren Veränderung (Levelierung: Erhöhung / Erniedrigung) in mindestens einer Patientenprobe kann die Diagnose erfolgen.

In einer weiteren Ausführungsform der Erfindung kann das erfindungsgemäße Verfahren im Rahmen einer in-vitro Diagnose mittels parallelen oder simultanen Bestimmungen der erfindungsgemäßen Marker durchgeführt werden (z.B. Multititerplatten mit 96 und mehr Kavitäten), wobei die Bestimmungen an mindestens einer Patientenprobe durchgeführt werden.

In einer weiteren Ausführungsform der Erfindung kann das erfindungsgemäße Verfahren mit Hilfe einer 2D-Elektrophorese erfolgen, wobei in der ersten Dimension eine isoelektrische Fokussierung, in der zweiten Dimension eine Gelelektrophorese durchgeführt wird (im weitesten Sinne ist hierzu die Proteomforschung ("Proteomics") anzuwenden).

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung.

In einer weiteren Ausführungsform kann das erfindungsgemäße Verfahren in-vivo durchgeführt werden, wobei die erfindungsgemäßen Biomarker mit einer Sonde, insbesondere ein Antikörper, die ein Kontrastmittel aufweisen markiert und mit einem in der Bildgebung geeignetem Detektor ("Molecular Imaging") nachgewiesen werden (Ralph Weissleder, Molecular Imaging in Cancer, Science, Vol. 312, 1168 (2006)).

Ferner betrifft die Erfindung die Verwendung der erfindungsgemäßen Biomarker zur Diagnose und / oder Prognose und/oder zur Früh- oder Differentialdiagnose von Hirntumoren, insbesondere Gliomen.

Die Erfindung betrifft zudem die Verwendung eines Kits zur Durchführung der erfindungsgemäßen Verfahren, insbesondere enthaltend Nachweisreagenzien und weitere Hilfsmittel. Solche Nachweisreagenzien umfassen z.B. Antikörper etc.

Der Nachweis und die Quantifizierung der erfindungsgemäßen Biomarker kann ebenfalls mit Hilfe weiterer, dem Fachmann geläufiger Protein-Diagnoseverfahren durchgeführt werden, insbesondere unter Verwendung radioaktiv oder fluoreszenzmarkierter Antikörper. Zu nennen sind hier insbesondere dazu geeignete bioanalytische Verfahren, wie zum Beispiel Immunhistochemie, Antikörperarrays, Luminex, ELISA, Immunfluoreszenz, Radioimmunoassays sowie weiteren geeigneten bioanalytischen Verfahren, wie zum Beispiel massenspektrometrische Verfahren, z.B. MRM (Multi reaction monitoring) oder AQUA (absolute Quantification), mit deren Hilfe die Biomarker quantitativ gemessen werden können, durchgeführt werden.

Nachfolgende Beispiele und Abbildungen dienen zur näheren Erläuterung der Erfindung, jedoch ohne die Erfindung auf diese Beispiele und Abbildungen zu beschränken.

### Beispiele und Abbildungen:

### Tumorproben:

Die Tumorproben wurden aus der Tumorbank des Institutes für Neuropathologie der Heinrich-Heine Universität Düsseldorf erhalten. Die Proben wurden nach den Kriterien der WHO Klassifikation für Tumore des Nervensystems (Kleihues et al., 2002 (supra)) eingeordnet und ein Tumorzellgehalt von mindestens 80% als zusätzliches Kriterium festgelegt.

In der Proteomanalyse wurden neun Tumorproben aus primären humanen Gliomen verwendet (5 x RET, 4 x LOH) und zur Validierung von Kandidatenproteinen weitere 51 der WHO-Grade II und III.

### Bestimmung des 1p/19q LOH Status:

Der LOH Status aller eingeschlossenen Tumorproben wurde mit bis zu 30 Microsatelliten auf Chromosom 1p und bis zu 5 auf dem Chromosomenarm 19q untersucht (Felsberg et al., 2004 (supra)).

### Probenvorbereitung und Proteinmarkierung für die 2D-DIGE Analyse:

Zur Zell-Lyse von solidem Tumormaterial wurde eine entsprechende Menge Tumor mit Lyse Puffer versetzt und zunächst manuell mit einem Pistill und anschließend in einem Ultraschallbad lysiert. Anschließend erfolgte ein Zentrifugationsschritt zur Entfernung unlöslicher Zelltrümmer. Der Proteingehalt wurde mit dem Bio-Rad Protein Assay (Bio-Rad, München, Germany) bestimmt. Zur differentiellen Analyse wurden je 50µg Protein mit 1µl Fluoreszenzfarbstoff nach Herstellerangaben markiert. Tumorproben mit LOH wurden mit Cy3, solche ohne LOH mit Cy5 markiert. Ein interner Standard, bestehend aus gleichen Teilen aller Proben der Analyse wurde mit Cy2 markiert. Für eine Identifizierung differentiell regulierter Proteine mittels MALDI-MS wurde ein präparatives Gel mit 400µg Protein erstellt. Die Gele wurden anschließend nachgefärbt und zur Dokumentation getrocknet (Nesterenko, M. V., Tilley, M., and Upton, S. J. (1994) A simple modification of Blum's silver stain method allows for 30 minute detection of proteins in polyacrylamide gels. J. Biochem. Biophys. Methods 28, 239-242).

### Großgel-Elektrophorese:

Die Analyse wurde mit modifizierter Carrier Ampholyte basierter isoelektrischen Fokussierung in selbst gegossenen 40cm Röhrchengelen mit einem Durchmesser von 1,5 mm durchgeführt (Klose, J. (1999) Large-gel 2-D electrophoresis. Methods Mol. Biol. 112, 147-172). Anschließend wurden die Gele ausgestoßen, äquilibriert und danach auf selbst hergestellte (40x30cm) 2D-Gele (SDS-PAGE) gelegt und dort mit Agarose fixiert.

### Bildakquisition und -analyse:

Für die Bildakquisition mit einem Typhoon Trio™ Fluorescence Imager (GE Healthcare) bleiben die Gele zwischen den Glasplatten. Die Anregungswellenlänge und die Emissionsfilter wurden spezifisch für die jeweiligen Fluoreszenzfarbstoffe gemäß dem Handbuch ausgewählt. Die generierten Bilder wurden vor der Bildanalyse mit der ImageQuant TL™ software (Version 2003.02, GE Healthcare) zugeschnitten. Intra-gel Spotdetektion und inter-gel Vergleich wurden mit den Modulen Differential In-gel Analysis (DIA) und Biological Variation Analysis (BVA) der DeCyder 6.0™ software (GE Healthcare) durchgeführt. Zur Standardisierung und Normalisierung der Spotintensitäten wurde der interne Standard benutzt. Differentiell regulierte Proteine wurden durch Student's t test bestimmt, und alle Proteine mit einem p-Wert ≤ 0.05 wurden für eine Identifikation mit MALDI-MS ausgewählt.

### Proteinidentifizierung mit MALDI-TOF/TOF MS:

Die erfindungsgemäßen Proteine wurden manuell aus dem Gel ausgeschnitten und anschließend im Gel mit Trypsin (Promega, Madison, WI) verdaut und für die Massenspektrometrie extrahiert (Marcus K, Immler D, Sternberger J, et al. Identification of platelet proteins separated by twodimensional gel elctrophoresis and analyzed by matrix assisted laser desorption/ionization-time of flight-mass spectrometry and detection of tyrosine-phosphorylated proteins. Electrophoresis, 2000;21,2622-2636). Die tryptischen Peptide eines jeden Proteins wurden mittels MALDI-TOF MS an einer UltraFlex II™ (Bruker Daltonics, Bremen, Germany) analysiert. Zur Präparation eines MALDI AnchorChip™ target (Bruker, Bremen, Deutschland) wurde nach Herstellerempfehlung α-Cyano-4-hydroxy-Zimtsäure verwendet. Die Spektren wurde im Positivmodus mit einer Target Spannung von 20kV samt einer gepulsten Ionen Extraktion von 17,25 kV. Die Reflektorspannung wurde auf 21 kV und die Detektorspannung auf 1,7 kV gesetzt. Die erhaltenen PMF (Peptide Mass Fingerprint) Spektren wurden mit einem Peptidmix kalibriert (Monoisotopisch Massen Bradykinin(1-7) m/z 757.399, Angiotensin I m/z 1296.685, Bombesin m/z 1619.822, ACTH clip(1-17) 2093.086 m/z and Somatostatin 3147.471 m/z). Die PMF Spektren wurden mit der FlexAnalysis™ software (Version 2.4, Bruker, Bremen, Deutschland) prozessiert. Uninterpretierte Spektren wurden mit der humanen IPI database (Version v3.15), unter Verwendung des Mascot (Matrix Science, London, UK) Algorithmus in ProteinScape™ (Bruker, Bremen, Deutschland) interpretiert. Die Datenbanksuchen wurden unter Annahme folgender Parameter durchgeführt: Feste Cystein Modifikation mit Propionamid, variable Modifikation als Folge von Methionin Oxidation, eine verfehlte Schnittstelle im Fall von nicht kompletter Trypsin Hydrolyse, einen Massentoleranz von 50ppm und ohne genaue Beschreibung des Molekulargewichts und des isoelektrischen Punktes der aus der 2D-PAGE erhaltenen Proteine. Identifizierte erfindungsgemäße Proteine wurden akzeptiert, wenn sie einen Mascot Wert von mindestens 67 aufwiesen.

### Western Blot Analyse:

Zusätzlich zu den Proben aus solidem Tumorgewebe aus dem 2D-DIGE Ansatz, wurden Proteinfraktionen, die während der Mikrosatellitenanalyse (DNA Gewinnung mittels Ultrazentrifugation in einem Guanidinhydrochlorid-Gradienten) angefallen sind (van den Boom, J., Wolter, M., Kuick, R., Misek, D. E., Youkilis, A.S., Wechsler, D. S., Sommer, C., Reifenberger, G., and Hanash, S. M. (2003) Characterization of gene expression profiles associated with glioma progression using oligonucleotide-based microarray analysis and real-time reverse transcription-polymerase chain reaction. Am J Pathol., 2003;163, 1033-1043), verwendet. Ein Überschuß an Guanidinhydrochlorid wurde entfernt und mit Lösemittel aus Probenpuffer mit Hilfe von Microcons™ (Millipore, 3kDa Cut-off) ersetzt. Die Proteine wurden auf vorgefertigten NuPAGE™ Novex 4-12% Bis-Tris Midi Gelen (Invitrogen Karlsruhe, Germany), 20 Taschen und 1,0 mm Dicke, aufgetrennt. Anschließend wurden die Proteine mit einem NovaBlot™ Blot Modul (GE Healthcare) auf eine Amersham Hybond™-PVDF Membran (GE Healthcare) übertragen. Unspezifische Bindungen und Wechselwirkungen der Membran wurden mit 3% (w/v) Milchpulver, 1% BSA und 0.1% (v/v) Tween 20 in Tris gepufferter Salzlösung, pH 8,5, für 1 Stunde bei Raumtemperatur abgeblockt. Die Immunoblots wurden dann über Nacht bei 4°C mit den primären Antikörpern in den entsprechenden Konzentrationen inkubiert und zwar ein Vilin2 Antikörper (Kaninchen, anti-human, 1:5000) von Biomol (Hamburg, Germany), ein Vimentin Antikörper (Maus, anti-human, 1:5000) von Acris Antibodies (Hiddenhausen, Germany), ein Annexin1 Antikörper (Maus, anti-human, 1:1000) (BD Transduction Laboratories (Heidelberg, Germany)) und ein Beta-Tubulin Antikörper zur Ladungskontrolle (Maus, anti-human, 1:2000) wurde bei Sigma (Seelze, Germany) verwendet. Die Membranen wurden 3x15min. gewaschen, für 1 Stunde bei Raumtemperatur mit den entsprechenden sekundären Antikörpern versehen und danach noch einmal 3x10min gewaschen. Die Immunkomplexe wurden mit dem Amersham ECL Plus™ Western Blotting Detection System (GE Healthcare) sichtbar gemacht.

### Immunhistochemie und statistische Analyse:

Für die immunhistochemische Analyse wurden 43 in Formalin fixierte und in Paraffin eingebettete Schnitte verwendet. 2,5µm Dicke Schnitte wurden auf poly-L-Lysin beschichtete Objektträger aufgebracht und über Nacht bei 37°C getrocknet. Nach der Deparaffinisierung, Rehydrierung und Zerstörung endogener Peroxidasen wurde die Antigen-Demaskierung in Natriumcitrat Puffer (pH6, 10mmol/l) für 15 bis 20 min. in der Mikrowelle durchgeführt. Die primären Antikörper gegen Annexin1 (1:100) und gegen Vilin2 (1:150) entsprachen denen der Western Blot Analyse. Der Vimentin Antikörper (1:200) wurde von BioGenex (San Ramon, USA) bezogen und der GFAP Antikörper (1:8000) von DAKO (Hamburg, Germany). Die Immunreaktivität wurde mit dem DAKO EnVision™ Detection System, HRP (DAKO, Hamburg, Germany) unter Verwendung von 3,3'-Diaminobenzidintetrahydrochlorid (DAB) als Chromogen durchgeführt. Zuletzt wurden alle Schnitte mit Hematoxylin leicht gegengefärbt. Jeder einzelne immunhistochemische Assay wurde mit entsprechenden Negativ- und externen Positivkontrollen durchgeführt.

Die Expressionsniveaus der einzelnen Proteine wurden in einen numerischen Wert, basierend auf dem Anteil der gefärbten Tumorzellen, umgewandelt. Dieser Index wurde in 5 Gruppen eingeteilt: 0 (keine oder minimale Reaktivität, weniger als 1% gefärbter Tumorzellen), 1 (kleiner 10% positiver Tumorzellen), 2 (10-50% positive Tumorzellen), 3 (50-90% positive Tumorzellen) und 4 (mehr als 90% gefärbte Tumorzellen. Die Schnitte wurden blind von 2 Personen unabhängig bewertet. Zur Illustration der Ergebnisse wurden Box-Whisker Plots erstellt und um einen statistisch signifikanten Unterschied der Expressionsunterschiede basierend auf dem LOH Status zu belegen wurde ein Mann-Whitney-U-Test benutzt (Statistica Software Version 7.1, Statsoft, Tulsa, USA). p-Werte ≤0,05 wurden als signifikant angesehen. Als Maß für die diagnostische Genauigkeit des Assays in der Vorhersage von Fällen mit und ohne LOH anzugeben wurde für jeden einzelnen immunhistochemischen Assay eine Receiver operation characteristics ("ROC") Kurve erstellt. Um die diagnostische Genauigkeit zu quantifizieren wurde für jedes Protein die Fläche unter der Kurve ("AUC") berechnet.

### Einfluss der erfindungsgemäßen Biomarker auf die Chemoresistenz von Glioblastomzelllinien

Alle erfindungsgemäßen Biomarker (SEQ ID No. 1 - 24), die in den differentiellen Untersuchungen identifiziert wurden, sind potentiell in der Lage an der Vermittlung der Chemosensitivität dieser Gliome beteiligt zu sein. Durch den Einsatz ausgewählter Zytostatika mit unterschiedlichen Wirkmechanismen kann überprüft werden, ob die erfindungsgemäßen Biomarker (SEQ ID No. 1 - 24) direkt mit dem Reaktionsmechanismus der in der Therapie üblichen Zytostatika wechselwirken. Hierzu wurden stabile Zelllinien hergestellt und die erfindungsgemäßen Biomarker verstärkt exprimiert (Überexpression) und vermindert (knock-down) exprimiert (siehe J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Eingesetzte Zytostatika: Carmustin und Vincristin, zwei Substanzen aus dem in der Therapie üblichen PCV Polychemotherapieschema, und Temozolomid, einem in der Therapie niedrig maligner Gliome neueren Wirkstoff. Zur Auswertung eines Effekts wurden Dosis-Wirkungs-Kurven und deren Lage zueinander und der korrespondierende EC50 Wert (Effektkonzentration; EC-50-Wert besagt, dass bei dieser Konzentration des Stoffes die Hälfte der Versuchsorganismen Wirkungen zeigt) berechnet.

### Behandlung der humanen Glioblastomzelllinie A172 mit Carmustin

**Tabelle 1: Chemosensitivität der humanen Glioblastomzelllinie A172 nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Carmustin. n.b, nicht bestimmt. KI, Konfidenzintervall.**

| **A172 Überexpression** | **EC₅₀ (µM)** | **95 % KI (µM)** | **p-Wert** |
|---|---|---|---|
| Kontrolle | 7,87 | 7,31-8,48 | |
| ANXA1 | 88,29 | 84,45-92,31 | **< 0.0001** |
| CLIC1 | 105,2 | 88,39-125,30 | **< 0.0001** |
| PRDX1 | 35,69 | 23,89-53,33 | **< 0.0001** |
| VIL2 | 106,1 | 93,29-120,70 | **< 0.0001** |

| **A172 Knock-Down** | **EC₅₀ (µM)** | **95 % KI (µM)** | **p-Wert** |
|---|---|---|---|
| Kontrolle | 94,4 | 87,8-101,5 | |
| ANXA1 | 95,6 | 91,9-99,4 | 0,7573 |
| CLlC1 | 170,6 | 152,9-190,4 | **< 0.0001** |
| PRDX1 | 106,6 | 96,3-118,0 | n.b. |
| VIL2 | 90,8 | 80, 9-101,9 | 0,5701 |

| | | | |
|---|---|---|---|
| p < 0,0001 ist signifikant | | | |

### Behandlung der humanen Glioblastomzelllinie A172 mit Temozolomid

**Tabelle 2: Chemosensitivität der humanen Glioblastomzelllinie A172 nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Temozolomid. KI, Konfidenzintervall.**

| **A172 Überexpression** | **EC₅₀ (µM)** | **95 % KI (µM)** | **p-Wert** |
|---|---|---|---|
| Kontrolle | 164,8 | 148,6-182,7 | |
| ANXA1 | 123,4 | 115,4-132,0 | **<0,0001** |
| CLlC1 | 82,6 | 64,3-106,2 | **<0,0001** |
| PRDX1 | 26,4 | 17,6-39,6 | **<0,0001** |
| VIL2 | 83,1 | 69,9-98,7 | **<0,0001** |

| **1A172 Knock-Down** | **EC₅₀ (µM)** | **95 % KI (µM)** | **p-Wert** |
|---|---|---|---|
| Kontrolle | 41,0 | 24,8-68,1 | |
| ANXA1 | 113,0 | 70,7-180,5 | **0,0076** |
| CLIC1 | 150,0 | 124,1-181,2 | **< 0.0001** |
| PRDX1 | 117,8 | 104,5-132,7 | **< 0.0001** |
| VIL2 | 114,9 | 97,4-135,5 | **< 0.0001** |

| | | | |
|---|---|---|---|
| p < 0,0001 ist signifikant | | | |

### Behandlung der humanen Glioblastomzelllinie A172 mit Vincristin

**Tabelle 3: Chemosensitivität der humanen Glioblastomzelllinie A172 nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Vincristin. n.b, nicht bestimmt. KI, Konfidenzintervall.**

| **A172 Überexpression** | **EC₅₀ (nM)** | **95 % KI (nM)** | **p-Wert** |
|---|---|---|---|
| Kontrolle | 15,2 | 12,8-18,0 | |
| ANXA1 | 15,3 | 11,4-20,5 | 0,9761 |
| CLIC1 | 19,3 | 16,5-22,6 | **0,0375** |
| PRDX1 | 12,7 | 10,1-15,8 | 0,1821 |
| VIL2 | 3,3 | 3,0-3,5 | **< 0.0001** |

| **A172 Knock-Down** | **EC₅₀ (nM)** | **95 % KI (nM)** | **p-Wert** |
|---|---|---|---|
| Kontrolle | 32,2 | 26,6-39,1 | |
| ANXA1 | 37,0 | 24,1-57,0 | 0,6242 |
| CLIC1 | 64,2 | 58,6-70,3 | n.b. |
| PRDX1 | 52,7 | 47,2-58,8 | **0,0042** |
| VIL2 | 20,2 | 15,5-26,3 | **0,0070** |

| | | | |
|---|---|---|---|
| p < 0,0001 ist signifikant | | | |

### Behandlung der humanen Glioblastomzelllinie T98G mit Carmustin

**Tabelle 4: Chemosensitivität der humanen Glioblastomzelllinie T98G nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Carmustin. n.b, nicht bestimmt. KI, Konfidenzintervall.**

| **T98G Überexpression** | **EC₅₀ (µM)** | **95 % KI (µM)** | **p-Wert** |
|---|---|---|---|
| Kontrolle | 64,8 | 49,4-85,2 | |
| ANXA1 | 206,7 | 192,1-222,4 | **< 0.0001** |
| CLIC1 | 346,3 | n.b. | **< 0.0001** |
| PRDX1 | 158,5 | 142,2-176,6 | **< 0.0001** |
| VIL2 | 223,1 | 210,7-236,1 | **< 0.0001** |

| **T98G knock-Down** | **EC₅₀ (µM)** | **95 % KI (µM)** | **p-Wert** |
|---|---|---|---|
| Kontrolle | 151,6 | 138,6-165,8 | |
| ANXA1 | 121 | 104,7-139,8 | **0,0069** |
| CLIC1 | 85,5 | 66,2-110,5 | **< 0.0001** |
| PRDX1 | 118,2 | 97,9-142,6 | **0,0207** |
| VIL2 | 159,4 | 147,6-172,2 | 0,4151 |

| | | | |
|---|---|---|---|
| p < 0,0001 ist signifikant | | | |

### Behandlung der humanen Glioblastomzelllinie T98G mit Temozolomid

**Tabelle 5: Chemosensitivität der humanen Glioblastomzelllinie T98G nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Temozolomid. KI, Konfidenzintervall.**

| **T98G Überexpression** | **EC₅₀ (µM)** | **95 % KI (µM)** | **p-Wert** |
|---|---|---|---|
| Kontrolle | 394,5 | 383,2-406,0 | |
| ANXA1 | 408,7 | 319,2-523,1 | 0,6967 |
| CLIC1 | 333,8 | 295,8-376,9 | **0,0289** |
| PRDX1 | 321,1 | 282,0-365,6 | **0,0476** |
| VIL2 | 395,7 | 359,4-435,7 | 0,9484 |

| **T98G knock-Down** | **EC₅₀ (µM)** | **95 % KI (µM)** | **p-Wert** |
|---|---|---|---|
| Kontrolle | 326,7 | 310,9-343,2 | |
| ANXA1 | 386,5 | 375,7-397,5 | **0,0012** |
| CLIC1 | 334,7 | 321,4-348,6 | 0,4104 |
| PRDX1 | 382,7 | 368,5-397,5 | 0,0525 |
| VIL2 | 347,6 | 331,4-364,6 | 0,1185 |

| | | | |
|---|---|---|---|
| p < 0,0001 ist signifikant | | | |

### Behandlung der humanen Glioblastomzelllinie T98G mit Vincristin

**Tabelle 6: Chemosensitivität der humanen Glioblastomzelllinie T98G nach modifizierter Expression von ANXA1 (SEQ ID No. 4), CLIC1 (SEQ ID No. 6), PRDX1 (SEQ ID No. 15 oder 16) und VIL2 (SEQ ID No. 9), gegenüber einer Behandlung mit Vincristin. KI, Konfidenzintervall.**

| **T98G Überexpression** | **EC₅₀ (nM)** | **95 % KI (nM)** | **p-Wert** |
|---|---|---|---|
| Kontrolle | 32,4 | 27,3-38,4 | |
| ANXA1 | 4,2 | 3,5-5,0 | **< 0.0001** |
| CLIC1 | 3,7 | 2,9-4,7 | **< 0.0001** |
| PRDX1 | 9,6 | 8,4-10,9 | **< 0.0001** |
| VIL2 | 24,1 | 19,0-30,8 | **0,0475** |

| **T98G knock-Down** | **EC₅₀ (nM)** | **95 % KI (nM)** | **p-Wert** |
|---|---|---|---|
| Kontrolle | 10,0 | 7,6-13,3 | |
| ANXA1 | 19,1 | 15,5-23,7 | **0,0009** |
| CLIC1 | 26,9 | 22,4-32,3 | **< 0.0001** |
| PRDX1 | 20,6 | 17,2-24,8 | **< 0,0001** |
| VIL2 | 35,8 | 30,1-42,5 | **< 0.0001** |

| | | | |
|---|---|---|---|
| p < 0,0001 ist signifikant | | | |

### Reaktion von Glioblastomzelllinien auf die Behandlung mit verschiedenen Zytostatika: Vergleich der verwendeten Zelllinien aus A172 und T98G

Eine veränderte Reaktion auf eine Behandlung mit Carmustin wurde in allen stabilen Zelllinien aus A172 sowie aus T98G beobachtet. Wurden die erfindungsgemäßen Biomarker überexprimiert, so wurde eine erhöhte EC₅₀ Werte gemessen. Nach *knock-down* der erfindungsgemäßen Biomarker wurden verminderte EC₅₀ Werte berechnet.

Nach einer Behandlung mit Temozolomid konnte bei einer Überexpression erfindungsgemäßen Biomarker in den stabilen Zelllinien aus A172 und T98G eine erhöhte Sensitivität gemessen werden. Nach Behandlung der *knock-down* Zelllinien wurde ein umgekehrter Effekt beobachtet.

Einen ähnlichen Effekt zeigte die Behandlung der Zelllinien mit Vincristin. In den Zelllinien, die die erfindungsgemäßen Biomarker überexprimierten, kam es zu einer Verschiebung zu niedrigeren Konzentrationen. Bei einem *knock-down* der erfindungsgemäßen Biomarker waren im Gegensatz dazu höhere Konzentrationen notwendig. Dieses Verhalten wurde ebenfalls in den beiden Zelllinien beobachtet.

Diese Ergebnisse zeigen ebenfalls, dass die erfindungsgemäßen Biomarker zur Durchführung von klinischen Entscheidungen und Therapiesteuerung geeignet sind.

### Abbildungen:

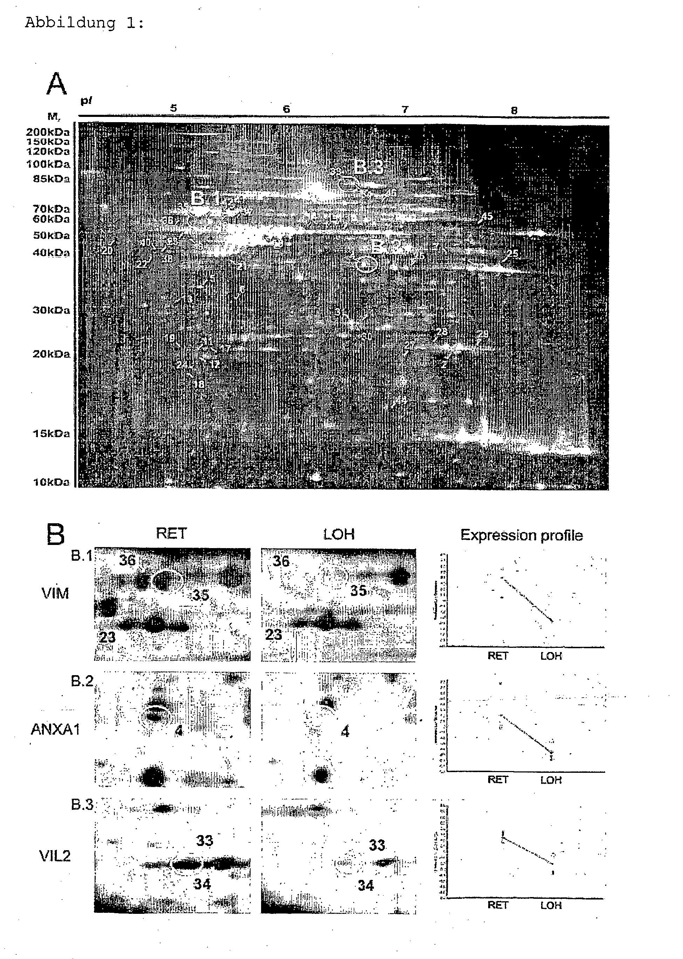

Abbildung 1A beschreibt ein repräsentatives großes (40x30cm) 2D-Gel mit ca. 3500 Proteinspots. Die Pfeile beziehen sich auf die entsprechenden identifizierten Proteine und erfindungsgemäßen Marker aus Tabelle 1.

Abbildung 1B beschreibt repräsentative Expressionsprofile von 4 differentiell regulierten Proteinen.

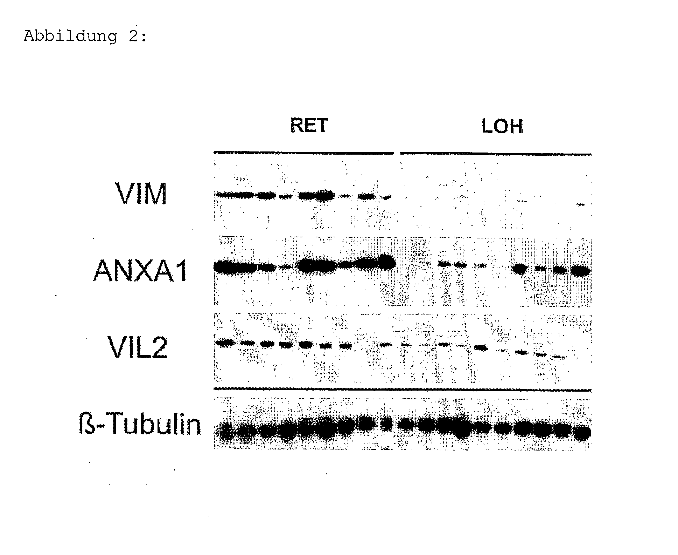

Abbildung 2 beschreibt die Ergebnisse der Western Blot Analyse für die Proteine Vimentin, Annexin1 und Villin2. Als Ladungskontrolle wurde ß-Tubulin benutzt.

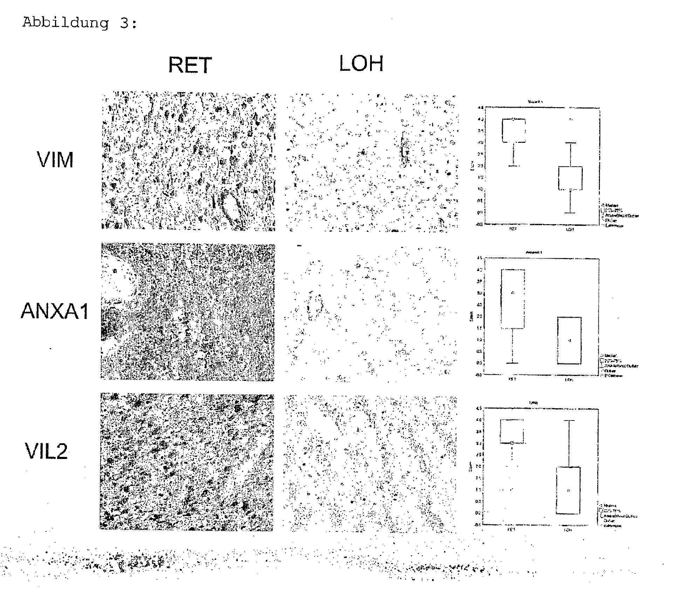

Abbildung 3 beschreibt repräsentative immunhistochemische Färbungen von Vimentin (VIM), Annexin1 (ANXA1) und Vilin2 (VIL2) von Tumorgewebe (Paraffin eingebettet) in 40-facher Vergrößerung. Die Ergebnisse der Färbungen von 43 Tumorproben werden mittels BOX-Whisker-Plots beschrieben.

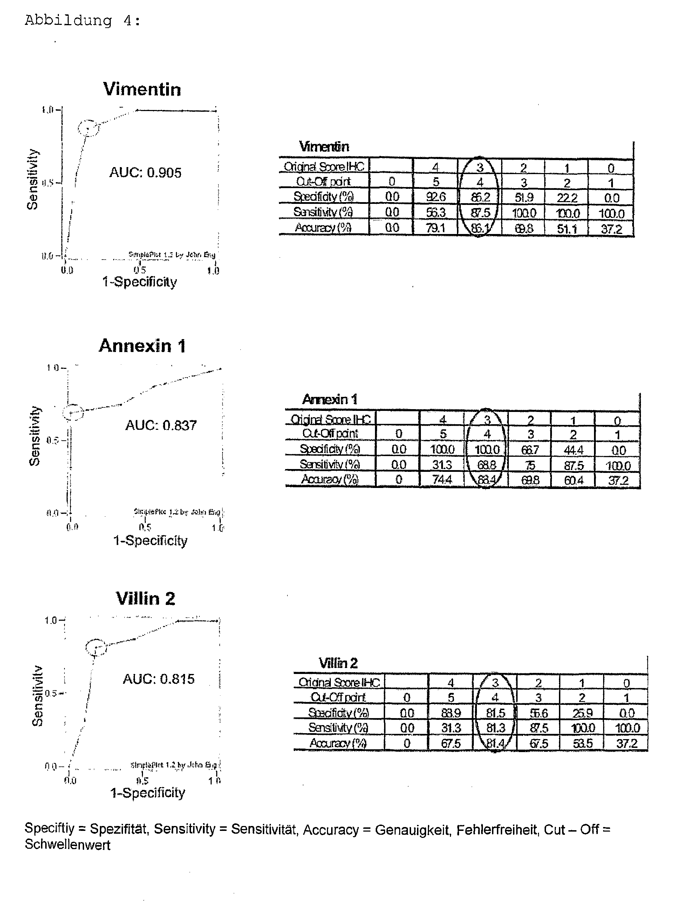

Abbildung 4 beschreibt die ROC (Receiver operation characteristics) Kurven von Vimentin mit einer AUC (Area under the curve) von 0.91, Annexin1 mit einer AUC von 0.84 und Vilin2 mit einer AUC von 0.82.

## Patentansprüche

1. Verfahren zur in-vitro Diagnose und / oder Risikostratifizierung von Hirntumoren, insbesondere Gliomen, wobei eine Bestimmung von Aldose reductase (SEQ ID No. 1, Acc. No. IPI00413641.6) von einem zu untersuchenden Patienten erfolgt, oder eine Bestimmung von Aldose reductase (SEQ ID No. 1, Acc. No. IPI00413641.6) in Kombination mit mindestens einem Biomarker ausgewählt aus der Gruppe
Alpha crystallin B chain (SEQ ID No. 2, Acc. No. IPI00021369.1),
Alpha-enolase (SEQ ID No. 3, Acc. No. IPI00465248.4),
Annexin A1 (SEQ ID No. 4, Acc. No. IPI00218918.4),
Annexin A5 (SEQ ID No. 5, Acc. No. IPI00329801.11),
Chloride Intracellular channel protein 1 (SEQ ID No. 6, Acc. No. IPI00010896.2),
DnaJ homolog subfamily B member 1 (SEQ ID No. 7, Acc. No. IPI00015947.4),
Gelsolin precursor (SEQ ID No. 8, Acc. No. IPI00026314.1),
Glial fibrillary acidic protein (SEQ ID No. 9, Acc. No. IPI00025362.1),
Glial fibrillary acidic protein (SEQ ID No. 10, Acc. No. IPI00443478.1),
Glial fibrillary acidic protein (SEQ ID No. 11, Acc. No. IPI00383815.4),
Glyceraldehyde-3-phosphate-dehydrogenase (SEQ ID No. 12, Acc. No. IPI00219018.6),
Heterogenous nuclear ribonucleoprotein (SEQ ID No. 13, Acc. No. IPI00477522.1),
Nucleosidediphosphate-linked moiety X motif 16 (SEQ ID No. 14, Acc. No. IPI00411732.4),
Peroxiredoxin-1 (SEQ ID No. 15, Acc. No. IPI00641244.1),
Peroxiredoxin-1 (SEQ ID No. 16, Acc. No. IPI00640741.1),
Peroxiredoxin-6 (SEQ ID No. 17, Acc. No. IPI00220301.4),
Sorbitoldehydrogenase (SEQ ID No. 18, Acc. No. IPI00216057.4),
Villin-2 (SEQ ID No. 19, Acc. No. IPI00479359.5),
Vimentin (SEQ ID No. 20, Acc. No. IPI00418471.5),
Vimentin (SEQ ID No. 21, Acc. No. IPI00552689.1),und
Peroxiredoxin-5 mitochondrialprecursor (SEQ ID No. 22, Acc. No. IPI00024915.2),
Platelet-activating factor acetylhydrolase IB alpha unit (SEQ ID No. 23, Acc. No. IPI00218728.2),
Syntaxin-bindung protein-1 (SEQ ID No. 24, Acc. No. IPI00641244.1),
von einem zu untersuchenden Patienten erfolgt.

2. Verfahren zur Diagnose und / oder Risikostratifizierung von Hirntumoren, insbesondere Gliomen, nach Anspruch 1, **dadurch gekennzeichnet, dass** eine Kombination der Biomarker nach Anspruch 1 zumindest Vimentin, Villin2 und / oder Annexin 1 enthält/enthalten.

3. Verfahren zur Diagnose und / oder Risikostratifizierung von Hirntumoren, insbesondere Gliomen, nach einem der Ansprüche 1 bis 2 zur Durchführung von klinischen Entscheidungen, insbesondere weiterführende Behandlung und Therapie mittels Arzneimitteln oder Chemotherapie.

4. Verfahren zur Diagnose und / oder Risikostratifizierung von Hirntumoren, insbesondere Gliomen, nach einem der Ansprüche 1 bis 3, **dadurch gekennzeichnet, dass** die Diagnose zur Prognose, zur differentialdiagnostischen Früherkennung und Erkennung, zur Beurteilung des Schweregrades und zur therapiebegleitenden Verlaufsbeurteilung erfolgt.

5. Verfahren nach einem der vorherigen Ansprüche,
**dadurch gekennzeichnet,**
**dass** parallele oder simultane Bestimmungen der Marker durchgeführt werden.

6. Verfahren nach einem der vorherigen Ansprüche,
**dadurch gekennzeichnet, dass** eine 2D-Elektrophorese erfolgt, wobei in der ersten Dimension eine isoelektrische Fokussierung, in der zweiten Dimension eine Gelelektrophorese durchgeführt wird.

7. Verfahren nach einem der vorherigen Ansprüche,
**dadurch gekennzeichnet,**
**dass** die Bestimmungen an mindestens einer Patientenprobe durchgeführt werden.

8. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

9. Verwendung eines Kits zur Diagnose nach einem der Ansprüche 1-8 enthaltend Nachweisreagenzien und weitere Hilfsmittel.

## Claims

1. A method for the *in vitro* diagnosis and/or risk stratification of brain tumours, in particular gliomas, wherein aldose reductase (SEQ ID No. 1, Acc. No. IPI00413641.6) is determined from a patient to be examined, or aldose reductase (SEQ ID No. 1, Acc. No. IPI00413641.6) in combination with at least one biomarker selected from the following group
alpha crystalline B chain (SEQ ID No. 2, Acc. No. IPI00021369.1),
alpha-enolase (SEQ ID No. 3, Acc. No. IPI00465248.4), annexin A1 (SEQ ID No. 4, Acc. No. IPI00218918.4),
annexin A5 (SEQ ID No. 5, Acc. No. IPI00329801.11), chloride intracellular channel protein 1 (SEQ ID No. 6, Acc. No. IPI00010896.2),
DnaJ homolog subfamily B member 1 (SEQ ID No. 7, Acc. No. IPI00015947.4),
gelsolin precursor (SEQ ID No. 8, Acc. No. IPI00026314.1),
glial fibrillary acidic protein (SEQ ID No. 9, Acc. No. IPI00025362.1),
glial fibrillary acidic protein (SEQ ID No. 10, Acc. No. IPI00443478.1),
glial fibrillary acidic protein (SEQ ID No. 11, Acc. No. IPI00383815.4),
glyceraldehyde-3-phosphate-dehydrogenase (SEQ ID No. 12, Acc. No. IPI00219018.6),
heterogenous nuclear ribonucleoprotein (SEQ ID No. 13, Acc. No. IPI00477522.1),
nucleosidediphosphate-linked moiety X motif 16 (SEQ ID No. 14, Acc. No. IPI00411732.4),
peroxiredoxin-1 (SEQ ID No. 15, Acc. No. IPI00641244.1),
peroxiredoxin-1 (SEQ ID No. 16, Acc. No. IPI00640741.1),
peroxiredoxin-6 (SEQ ID No. 17, Acc. No. IPI00220301.4),
sorbitoldehydrogenase (SEQ ID No. 18, Acc. No. IPI00216057.4),
villin-2 (SEQ ID No. 19, Acc. No. IPI00479359.5),
vimentin (SEQ ID No. 20, Acc. No. IPI00418471.5),
vimentin (SEQ ID No. 21, Acc. No. IPI00552689.1), and peroxiredoxin-5 mitochondrial precursor (SEQ ID No. 22, Acc. No. IPI00024915.2),
platelet-activating factor acetylhydrolase IB alpha unit (SEQ ID No. 23, Acc. No. IPI00218728.2),
syntaxin-binding protein-1 (SEQ ID No. 24, Acc. No. IPI00641244.1),
is determined from a patient to be examined.

2. The method for diagnosis and/or risk stratification of brain tumours, in particular gliomas, according to Claim 1, **characterised in that** a combination of the biomarkers according to Claim 1 contains/contain at least vimentin, villin-2 and/or annexin-1.

3. The method for diagnosis and/or risk stratification of brain tumours, in particular gliomas, according to one of Claims 1 to 2 for making clinical decisions, in particular continued treatment and therapy by means of drugs or chemotherapy.

4. The method for diagnosis and/or risk stratification of brain tumours, in particular gliomas, according to one of Claims 1 to 3, **characterised in that** the diagnosis is made for prognosis, for differential-diagnostic early diagnosis and diagnosis, for assessment of the degree of severity, and for the therapy-accompanying assessment of progress.

5. The method according to one of the preceding claims, **characterised in that**
the markers are determined in parallel or simultaneously.

6. The method according to one of the preceding claims, **characterised in that**
2D electrophoresis is carried out, wherein isoelectric focusing is carried out in the first dimension and gel electrophoresis is carried out in the second dimension.

7. The method according to one of the preceding claims, **characterised in that**
the determinations are performed on at least one patient sample.

8. The method according to one of the preceding claims, **characterised in that** the determinations are carried out by means of a rapid test, in particular in individual parameter or multi-parameter determinations.

9. Use of a kit for diagnosis according to one of Claims 1-8, containing detection reagents and further additives.

## Revendications

1. Méthode de diagnostic pour le diagnostic *in vitro* et/ou la stratification du risque de tumeurs cérébrales, en particulier de gliomes, où l'aldose réductase (SEQ ID n° 1, Acc. n° IPI00413641.6) est dosée chez un patient à examiner, ou où l'aldose réductase (SEQ ID n° 1, Acc. n° IPI00413641.6), en combinaison avec au moins un biomarqueur choisi dans le groupe suivant :
chaîne B de l'alpha-crystalline (SEQ ID n° 2, Acc. n° IPI00021369.1),
alpha-énolase (SEQ ID n° 3, Acc. n° IPI00465248.4),
annexine A1 (SEQ ID n° 4, Acc. n° IPI00218918.4),
annexine A5 (SEQ ID n° 5, Acc. n° IPI00329801.11),
protéine de canal chlorure intracellulaire 1 (SEQ ID n° 6, Acc. n° IPI00010896.2),
membre 1 de la sous-famille B des homologues de DnaJ (SEQ ID n° 7, Acc. N° IPI00015947.4),
précurseur de gelsoline (SEQ ID n° 8, Acc. n° IPI00026314.1),
protéine acide fibrillaire gliale (SEQ ID n° 9, Acc. n° IPI00025362.1),
protéine acide fibrillaire gliale (SEQ ID n° 10, Acc. n° IPI00443478.1),
protéine acide fibrillaire gliale (SEQ ID n° 11, Acc. n° IPI00383815.4),
glycéraldéhyde-3-phosphate-déshydrogénase (SEQ ID n° 12, Acc. n° IPI00219018.6),
ribonucléoprotéine nucléaire hétérogène (SEQ ID n° 13, Acc. n° IPI00477522.1),
Nudix (*nucleosidediphosphate-linked moiety X*) motif 16 (SEQ ID n° 14, Acc. n° IPI00411732.4),
peroxirédoxine-1 (SEQ ID n° 15, Acc. n° IPI00641244.1),
peroxirédoxine-1 (SEQ ID n° 16, Acc. n° IPI00640741.1),
peroxirédoxine-6 (SEQ ID n° 17, Acc. n° IPI00220301.4),
sorbitol déshydrogenase (SEQ ID n° 18, Acc. n° IPI00216057.4),
villine-2 (SEQ ID n° 19, Acc. n° IPI00479359.5),
vimentine (SEQ ID n° 20, Acc. n° IPI00418471.5),
vimentine (SEQ ID n° 21, Acc. n° IPI00552689.1), et
précurseur mitochondrial de peroxirédoxine-5 (SEQ ID n° 22, Acc. n° IPI00024915.2),
unité alpha de la facteur d'activation des plaquettes-acétylhydrolase IB (SEQ ID n° 23, Acc. n° IPI00218728.2),
protéine se liant à la syntaxine-1 (SEQ ID n° 24, Acc. n° IPI00641244.1),
est dosée chez un patient à examiner.

2. Méthode de diagnostic et/ou de stratification du risque de tumeurs cérébrales, en particulier de gliomes, conforme à la Revendication 1, **caractérisée en ce qu'**une combinaison des biomarqueurs conformes à la Revendication 1 contient au moins la vimentine, la villine-2 et/ou l'annexine-1.

3. Méthode de diagnostic et/ou de stratification du risque de tumeurs cérébrales, en particulier de gliomes, conforme à l'une des Revendications 1 à 2 pour la prise de décisions cliniques, en particulier pour la poursuite d'un traitement et d'une thérapie par médicaments ou chimiothérapie.

4. Méthode de diagnostic et/ou de stratification du risque de tumeurs cérébrales, en particulier de gliomes, conforme à l'une des Revendications 1 à 3, **caractérisée en ce que** le diagnostic est réalisé à des fins de pronostic, de diagnostic différentiel, de diagnostic précoce et de diagnostic, d'évaluation du degré de gravité, et d'évaluation de l'évolution accompagnant la thérapie.

5. Méthode conforme à l'une des revendications précédentes, **caractérisée en ce que** :
les marqueurs sont dosés en parallèle ou simultanément.

6. Méthode conforme à l'une des revendications précédentes, **caractérisée en ce que** :
une électrophorèse 2D est mise en oeuvre, une focalisation isoélectrique étant mise en oeuvre dans la première dimension et une électrophorèse sur gel étant mise en oeuvre dans la deuxième dimension.

7. Méthode conforme à l'une des revendications précédentes, **caractérisée en ce que** :
les dosages sont mis en oeuvre sur au moins un échantillon de patient.

8. Méthode conforme à l'une des revendications précédentes, **caractérisée en ce que** les dosages sont mis en oeuvre par le biais d'un test rapide, en particulier dans les dosages de paramètres individuels ou multi-paramètres.

9. Emploi d'une trousse pour diagnostic conforme à l'une des Revendications 1 à 8, contenant des réactifs de détection et des adjuvants supplémentaires.
